(19) Europäisches Patentamt
European Patent Office
Office européen des brevets

(11) Veröffentlichungsnummer: **0 377 896**
**A2**

# (12) EUROPÄISCHE PATENTANMELDUNG

(21) Anmeldenummer: **89123976.6**

(51) Int. Cl.[5]: **C07C 259/06, A61K 31/165**

(22) Anmeldetag: **27.12.89**

Die Bezeichnung der Erfindung wurde geändert (Richtlinien für die Prüfung im EPA, A-III, 7.3).

(30) Priorität: **29.12.88 CH 4843/88**

(43) Veröffentlichungstag der Anmeldung: **18.07.90 Patentblatt 90/29**

(84) Benannte Vertragsstaaten: **AT BE CH DE ES FR GB GR IT LI LU NL SE**

(71) Anmelder: **CIBA-GEIGY AG**
**Klybeckstrasse 141**
**CH-4002 Basel(CH)**

(72) Erfinder: **Sallmann, Alfred, Dr.**
**Joachimsackerstrasse 12**
**CH-4103 Bottmingen(CH)**

(74) Vertreter: **Zumstein, Fritz, Dr. et al**
**Bräuhausstrasse 4**
**D-8000 München 2(DE)**

## (54) Cyclooxygenase- und 5-Lipoxygenase-inhibierende Hydroxansäurederivate.

(57) Verbindungen der Formel

$$Ar_1 - \underset{R_1}{N} - Ar_2 - Z - CO - \underset{R_2}{N} - O - R_3 \qquad (I),$$

worin $Ar_1$ einen unsubstituierten oder substituierten Arylrest bedeutet, $Ar_2$ einen unsubstituierten oder substituierten Arylenrest bedeutet, $R_1$ Wasserstoff oder einen aliphatischen oder araliphatischen Rest bedeutet, $R_2$ einen aliphatischen oder araliphatischen Rest bedeutet, $R_3$ Wasserstoff, Niederalkyl oder Niederalkanoyl darstellt und Z einen zweiwertigen aliphatischen Rest bedeutet, und ihre Salze sind in an sich bekannter Weise herstellbar. Sie können z.B. als Arzneimittelwirkstoffe verwendet werden.

EP 0 377 896 A2

Xerox Copy Centre

# Carbonsäurederivate

Die Erfindung betrifft neue Carbonsäurederivate der Formel

$$Ar_1 - \underset{R_1}{\underset{|}{N}} - Ar_2 - Z - CO - \underset{R_2}{\underset{|}{N}} - O - R_3 \qquad (I),$$

worin $Ar_1$ einen unsubstituierten oder substituierten Arylrest bedeutet, $Ar_2$ einen unsubstituierten oder substituierten Arylenrest bedeutet, $R_1$ Wasserstoff oder einen aliphatischen oder araliphatischen Rest bedeutet, $R_2$ einen aliphatischen oder araliphatischen Rest bedeutet, $R_3$ Wasserstoff, Niederalkyl oder Niederalkanoyl darstellt und Z einen zweiwertigen aliphatischen Rest bedeutet, und ihre Salze, ein Verfahren zur Herstellung dieser Verbindungen oder ihrer Salze, diese Verbindungen oder ihre pharmazeutisch verwendbaren Salze enthaltende pharmazeutische Präparate und die Verwendung dieser Verbindungen und ihrer Salze.

Aryl $Ar_1$ steht in erster Linie für Phenyl. Aryl $Ar_1$ kann ein- oder mehrfach, z.B. zwei-, drei- oder vierfach, substituiert sein, beispielsweise durch Substituenten ausgewählt aus der Gruppe bestehend aus Niederalkyl, Niederalkoxy, Halogen, Hydroxy, Trifluormethyl und Phenylniederalkoxy, wie 1-Phenylniederalkoxy, wobei in Phenylniederalkoxy Phenyl unsubstituiert oder ein- oder mehrfach, z.B. zwei- oder dreifach, substituiert, beispielsweise durch Substituenten ausgewählt aus der Gruppe bestehend aus Niederalkyl, Niederalkoxy, Halogen, Hydroxy und Trifluormethyl, sein kann. Vorzugsweise sind die Substituenten in substituiertem Aryl $Ar_1$ ausgewählt aus der Gruppe bestehend aus Halogen und Niederalkyl.

Arylen $Ar_2$ bedeutet in erster Linie Phenylen, d.h. 1,3- oder 1,4- oder bevorzugt 1,2-Phenylen, oder Thienylen, wie 2,3- oder bevorzugt 3,4-Thienylen, wobei $Ar_2$ ein- oder mehrfach, z.B. zwei- oder, im Fall von Phenylen, auch dreifach, substituiert sein kann, beispielsweise durch Substituenten ausgewählt aus der Gruppe bestehend aus Niederalkyl, Niederalkoxy, Halogen, Trifluormethyl und Hydroxy.

Ein aliphatischer Rest ($R_1$, $R_2$) ist insbesondere unsubstituiert und bedeutet vorzugsweise Niederalkyl oder Niederalkenyl, wobei die Doppelbindung insbesondere in höherer als der $\alpha$-Stellung lokalisiert ist.

Ein araliphatischer Rest ($R_1$, $R_2$) ist in erster Linie Phenylniederalkyl, bevorzugt 1-Phenylniederalkyl, wobei Phenyl unsubstituiert oder ein- oder mehrfach, z.B. zwei- oder dreifach, substituiert sein kann, beispielsweise durch Substituenten ausgewählt aus der Gruppe bestehend aus Niederalkyl, Niederalkoxy, Halogen, Hydroxy und Trifluormethyl. Bevorzugt sind entsprechende unsubstituierte Reste, wie Benzyl oder 1-Phenylethyl.

Ein zweiwertiger aliphatischer Rest Z ist insbesondere unsubstituiert und bedeutet in erster Linie Niederalkylen, Niederalkyliden oder Niederalkenylen, bevorzugt eine Gruppe der Formel $-(CH_2)_m-(CH=CH)_n-$, worin m und n unabhängig voneinander für die ganze Zahl 0 oder 1, nicht jedoch gleichzeitig für 0, stehen oder m für 2 oder 3 steht und n für 0 steht.

Die Verbindungen I können über ihre basischen Zentren Salze bilden. Salze von Verbindungen I sind daher insbesondere entsprechende Säureadditionssalze, vorzugsweise pharmazeutisch verwendbare Säureadditionssalze. Diese werden beispielsweise mit starken anorganischen Säuren, wie Mineralsäuren, z.B. Schwefelsäure, einer Phosphorsäure oder einer Halogenwasserstoffsäure, mit starken organischen Carbonsäuren, wie Niederalkancarbonsäuren, z.B. Essigsäure, wie gegebenenfalls ungesättigten Dicarbonsäuren, z.B. Malon-, Malein- oder Fumarsäure, oder wie Hydroxycarbonsäuren, z.B. Wein- oder Zitronensäure, oder mit Sulfonsäuren, wie Niederalkan- oder gegebenenfalls substituierten Benzolsulfonsäuren, z.B. Methan- oder p-Toluolsulfonsäure, gebildet. Verbindungen I mit sauren Zentren, z.B. solche Verbindungen I, worin $R_3$ Wasserstoff ist, können Salze mit Basen bilden, wobei pharmazeutisch verwendbare Salze mit Basen bevorzugt sind. Geeignete Salze mit Basen sind bei spielsweise entsprechende Alkalimetall- oder Erdalkalimetallsalze, z.B. Natrium-, Kalium- oder Magnesiumsalze, oder pharmazeutisch verwendbare Uebergangsmetallsalze, wie Zink- oder Kupfersalze.

Umfasst sind ferner für pharmazeutische Verwendungen ungeeignete Salze, da diese beispielsweise für die Isolierung bzw. Reinigung freier Verbindungen I sowie deren pharmazeutisch verwendbarer Salze verwendet werden können.

Die Erfindung betrifft beispielsweise Verbindungen I, worin $Ar_1$ einen unsubstituierten oder substituierten Arylrest bedeutet, $Ar_2$ einen unsubstituierten oder substituierten Arylenrest bedeutet, $R_1$ Wasserstoff oder einen aliphatischen oder araliphatischen Rest bedeutet, $R_2$ einen aliphatischen oder araliphatischen Rest bedeutet, $R_3$ Wasserstoff ist und Z einen zweiwertigen aliphatischen Rest bedeutet, ein Verfahren zur Herstellung dieser Verbindungen, diese Verbindungen enthaltende pharmazeutische Präparate und die

Verwendung dieser Verbindungen, wobei die erwähnten Verbindungen I jeweils in freier Form oder in Form von Salzen vorliegen können, mit der Massgabe, dass als Verbindungen I in Salzform, welche in pharmazeutischen Präparaten enthalten sind, Verbindungen I in Form von pharmazeutisch verwendbaren Salzen vorliegen.

Vor- und nachstehend sind unter mit "Nieder" bezeichneten Resten oder Verbindungen, sofern nicht abweichend definiert, solche zu verstehen, die bis und mit 7, vor allem bis und mit 4, Kohlenstoffatome (C-Atome) enthalten.

Niederalkyl ist Methyl, Ethyl, n-Propyl, Isopropyl, n-Butyl, Isobutyl, sek-Butyl oder tert-Butyl und umfasst ferner entsprechende Pentyl-, Hexyl- und Heptylreste.

Niederalkoxy ist Methoxy, Ethoxy, n-Propyloxy, Isopropyloxy, n-Butyloxy, Isobutyloxy, sek-Butyloxy oder tert-Butyloxy und umfasst ferner entsprechende Pentyloxy-, Hexyloxy- und Heptyloxyreste.

Halogen ist insbesondere Halogen mit einer Atomnummer bis und mit 35, d.h. Fluor, Chlor oder Brom, und umfasst ferner Iod.

Niederalkenyl ist geradkettig oder verzweigt, weist insbesondere 3 oder 4 C-Atome auf und bedeutet vorzugsweise Allyl oder But-2-enyl.

Niederalkylen ist geradkettig oder verzweigt, weist insbesondere bis und mit 4 C-Atome auf und bedeutet vorzugsweise Methylen, Ethylen, 1,3-, 1,2-oder 2,3-Propylen, 1,1- oder 2,2-Dimethyl-ethylen oder 1,4-Butylen.

Niederalkyliden ist geradkettig oder verzweigt, weist insbesondere 2 bis und mit 4 C-Atome auf und bedeutet vorzugsweise Ethyliden, 1,1- oder 2,2-Propyliden oder 1,1- oder 2,2-Butyliden.

Niederalkenylen ist geradkettig oder verzweigt, weist insbesondere 2 bis und mit 4 C-Atome auf und bedeutet vorzugsweise Ethenylen, 1,3-Prop-1-enylen, 1,3-Prop-2-enylen, 1,4-But-1-enylen, 1,4-But-2-enylen oder 1,4-But-3-enylen.

Niederalkanoyl ist $C_1$-$C_7$-Alkanoyl, vorzugsweise $C_2$-$C_5$-Alkanoyl, z.B. Formyl, Acetyl, Propionyl, n-Butyryl, Isobutyryl oder Pivaloyl.

Phenylniederalkyl ist Phenyl-$C_1$-$C_7$-alkyl, insbesondere Phenyl-$C_1$-$C_4$-alkyl, wobei sich die Phenylgruppe vorzugsweise in 1-Stellung der Alkylgruppe befindet, z.B. Benzyl, 1-Phenylethyl oder 1-Phenylpropyl; der Phenylring kann dabei wie vorstehend angegeben substituiert oder vorzugsweise unsubstituiert sein.

Phenylniederalkoxy ist Phenyl-$C_1$-$C_7$-alkoxy, insbesondere Phenyl-$C_1$-$C_4$-alkoxy, wobei sich die Phenylgruppe vorzugsweise in 1-Stellung der Alkoxygruppe befindet, z.B. Benzyloxy oder 1-Phenylethoxy; der Phenylring kann dabei wie vorstehend angegeben substituiert oder vorzugsweise unsubstituiert sein.

Die Verbindungen I und ihre pharmazeutisch verwendbaren Salze weisen wertvolle pharmakologische Eigenschaften auf. In entsprechenden Testmodellen erweisen sie sich z.B. als potente duale Hemmer von Cyclooxygenase und 5-Lipoxygenase.

Verbindungen mit Cyclooxygenase- oder 5-Lipoxygenase-inhibierenden Eigenschaften sind bereits bekannt. Das Ziel vielfacher Bemühungen besteht darin, wirksame Hemmer sowohl von Cyclooxygenase als auch von 5-Lipoxygenase ("duale Hemmer") zu finden.

Die Enzyme Cyclooxygenase und 5-Lipoxygenase spielen im Metabolismus von Arachidonsäure eine bedeutende Rolle. Während der durch Cyclooxygenase induzierte Metabolismus von Arachidonsäure unter anderem zur Bildung von Prostaglandinen, z.B. von Prostaglandin $E_2$ ($PGE_2$), und Thromboxanen, z.B. von Thromboxan $A_2$ ($TXA_2$), führt, werden im durch 5-Lipoxygenase induzierten Abbauweg Leukotriene, z.B. Leukotrien $B_4$ ($LTB_4$) und Leukotrien $C_4$ ($LTC_4$), gebildet. $PGE_2$ und $TXA_2$ wird Bedeutung bei der Entwicklung von entzündlichen und schmerzlichen Prozessen zugemessen. Dementsprechend lassen sich Hemmer von Cyclooxygenase zur Behandlung von entzündlichen Prozessen einsetzen. Die Leukotriene spielen eine wichtige Rolle bei der Entstehung von allergischen und entzündlichen Prozessen. Somit können 5-Lipoxygenasehemmer zur Behandlung von Allergien verwendet werden.

Die Hemmwirkung der Verbindungen I und ihrer pharmazeutisch verwendbaren Salze auf das Enzym Cyclooxygenase lässt sich experimentell an Hand der Inhibition der $PGE_2$-Bildung in durch Zymosan stimulierten Mäuse-Peritoneal-Makrophagen verifizieren, wobei $IC_{50}$-Werte zwischen etwa 0,07 und etwa 1,5 $\mu$M beobachtet werden.

Auch die Hemmwirkung auf das Enzym 5-Lipoxygenase kann für die Verbindungen I und ihre pharmazeutisch verwendbaren Salze experimentell bestätigt werden, beispielsweise an Hand der Hemmung der $LTC_4$-Bildung in durch Zymosan stimulierten Mäuse-Peritoneal-Makrophagen, wobei $IC_{50}$-Werte zwischen etwa 0,02 und etwa 1,0 $\mu$M beobachtet werden.

Diese Ergebnisse können auch ex vivo am Hund nach oraler Applikation bestätigt werden.

Die entzündungshemmenden Eigenschaften der Verbindungen I und ihrer pharmazeutisch verwendbaren Salze zeigen sich auch in den klassischen chronischen und subchronischen Entzündungsmodellen, z.B. an der experimentellen Adjuvans-Arthritis-Ratte.

Ebenfalls vorhandene hautphlogistatische Aktivitäten der Verbindungen I und ihrer pharmazeutisch verwendbaren Salze lassen sich in vivo an Hand der Hemmwirkung auf das experimentelle Croton-Ohroedem an der Maus nachweisen, wobei analog der Methodik von A. Tubaro et al., Agents and Actions 19, 371 (1986), vorgegangen wird.

Im Hinblick auf die vorstehend dargelegten pharmakologischen Eigenschaften können die Verbindungen I und ihre pharmazeutisch verwendbaren Salze z.B. als Wirkstoffe in Antiinflammatorika und Antiallergika verwendet werden, welche beispielsweise zur Behandlung von entzündlichen Prozessen, Allergien, z.B. allergischer Rhinitis, Asthma, z.B. Asthma bronchiale, und Anaphylaxis eingesetzt werden. Ein Gegenstand der Erfindung ist daher die Verwendung der Verbindungen I und ihrer pharmazeutisch verwendbaren Salze zur Herstellung entsprechender Arzneimittel. Dabei ist die gewerbliche Herrichtung der Wirksubstanzen eingeschlossen.

Die Erfindung betrifft in erster Linie Verbindungen der Formel I, worin $Ar_1$ unsubstituiertes oder durch Substituenten ausgewählt aus der Gruppe bestehend aus Niederalkyl, Niederalkoxy, Halogen, Hydroxy, Trifluormethyl und Phenylniederalkoxy, wobei in Phenylniederalkoxy Phenyl unsubstituiert oder ein- oder mehrfach durch Substituenten augewählt aus der Gruppe bestehend aus Niederalkyl, Niederalkoxy, Halogen, Hydroxy und Trifluormethyl substituiert ist, ein- oder mehrfach substituiertes Phenyl bedeutet, $Ar_2$ einen unsubstituierten oder ein- oder mehrfach durch Substituenten ausgewählt aus der Gruppe bestehend aus Niederalkyl, Niederalkoxy, Trifluormethyl, Halogen und Hydroxy substituierten Phenylen- oder Thienylenrest bedeutet, $R_1$ Wasserstoff, Niederalkyl, Niederalkenyl oder Phenylniederalkyl, welches im Phenylteil unsubstituiert oder durch Substituenten ausgewählt aus der Gruppe bestehend aus Niederalkyl, Niederalkoxy, Halogen, Hydroxy und Trifluormethyl ein- oder mehrfach substituiert ist, darstellt, $R_2$ Niederalkyl, Niederalkenyl oder Phenylniederalkyl, welches im Phenylteil unsubstituiert oder durch Substituenten ausgewählt aus der Gruppe bestehend aus Niederalkyl, Niederalkoxy, Halogen, Hydroxy und Trifluormethyl ein- oder mehrfach substi tuiert ist, bedeutet, $R_3$ Wasserstoff, Niederalkyl oder Niederalkanoyl darstellt und Z Niederalkylen, Niederalkyliden oder Niederalkenylen ist, und ihre Salze.

Die Erfindung betrifft insbesondere Verbindungen der Formel I, worin $Ar_1$ unsubstituiertes oder durch Substituenten ausgewählt aus der Gruppe bestehend aus Niederalkyl, Niederalkoxy, Halogen, Hydroxy, Trifluormethyl und Phenylniederalkoxy, insbesondere 1-Phenylniederalkoxy, wobei Phenyl unsubstituiert oder ein- oder mehrfach, z.B. zwei- oder dreifach, beispielsweise durch Substituenten ausgewählt aus der Gruppe bestehend aus Niederalkyl, Niederalkoxy, Halogen, Hydroxy und Trifluormethyl, substituiert sein kann, substituiertes Phenyl bedeutet, $Ar_2$ einen unsubstituierten oder durch Substituenten ausgewählt aus der Gruppe bestehend aus Niederalkyl, Niederalkoxy, Trifluormethyl, Halogen und Hydroxy substituierten Phenylen-, wie 1,2-Phenylen-, oder Thienylen-, wie 3,4-Thienylen-rest, bedeutet, $R_1$ Wasserstoff, Niederalkyl, Niederalkenyl oder Phenylniederalkyl, welches im Phenylteil unsubstituiert oder durch Substituenten ausgewählt aus der Gruppe bestehend aus Niederalkyl, Niederalkoxy, Halogen, Hydroxy und Trifluormethyl substituiert ist, darstellt, $R_2$ Niederalkyl, Niederalkenyl oder Phenylniederalkyl, welches im Phenylteil unsubstituiert oder durch Substituenten ausgewählt aus der Gruppe bestehend aus Niederalkyl, Niederalkoxy, Halogen, Hydroxy und Trifluormethyl substituiert ist, darstellt, $R_3$ Wasserstoff ist und Z Niederalkylen, Niederalkyliden oder Niederalkenylen bedeutet, in freier Form oder in Form von Salzen.

Die Erfindung betrifft vor allem Verbindungen der Formel I, worin $Ar_1$ unsubstituiertes oder durch Substituenten ausgewählt aus der Gruppe bestehend aus Niederalkyl mit bis und mit 7 C-Atomen, Niederalkoxy mit bis und mit 7 C-Atomen, Halogen mit einer Atomnummer bis und mit 35, Hydroxy, Trifluormethyl und 1-Phenylniederalkoxy mit bis und mit 4 C-Atomen im Niederalkoxyteil, wie Benzyloxy, substituiertes Phenyl bedeutet, $Ar_2$ einen unsubstituierten oder durch Substituenten ausgewählt aus der Gruppe bestehend aus Niederalkyl mit bis und mit 7 C-Atomen, Halogen mit einer Atomnummer bis und mit 35 und Hydroxy substituierten Phenylen- oder Thienylen-rest bedeutet, $R_1$ Wasserstoff, Niederalkyl mit bis und mit 7 C-Atomen, Niederalkenyl mit 3 oder 4 C-Atomen oder Phenyl niederalkyl, wie 1-Phenylniederalkyl, welches bis und mit 7 C-Atome im Niederalkylteil aufweist und dessen Phenylteil, wie 1-Phenylteil, unsubstituiert oder durch Substituenten ausgewählt aus der Gruppe bestehend aus Niederalkyl mit bis und mit 7 C-Atomen, Niederalkoxy mit bis und mit 7 C-Atomen, Halogen mit einer Atomnummer bis und mit 35, Hydroxy und Trifluormethyl substituiert ist, darstellt, $R_2$ Niederalkyl mit bis und mit 7 C-Atomen, Niederalkenyl mit 3 oder 4 C-Atomen oder Phenylniederalkyl, wie 1-Phenylniederalkyl, welches bis und mit 7 C-Atome im Niederalkylteil aufweist und dessen Phenylteil, wie 1-Phenylteil, unsubstituiert oder durch Substituenten ausgewählt aus der Gruppe bestehend aus Niederalkyl mit bis und mit 7 C-Atomen, Niederalkoxy mit bis und mit 7 C-Atomen, Halogen mit einer Atomnummer bis und mit 35, Hydroxy und Trifluormethyl substituiert ist, darstellt, $R_3$ Wasserstoff ist und Z Niederalkylen mit bis und mit 4 C-Atomen, Niederalkyliden mit 2 bis und mit 4 C-Atomen oder Niederalkenylen mit 2 bis und mit 4 C-Atomen bedeutet, in freier Form oder in Form von Salzen.

Die Erfindung betrifft bevorzugt Verbindungen der Formel I, worin $Ar_1$ durch Niederalkyl mit bis und mit 4 C-Atomen, wie Methyl, Niederalkoxy mit bis und mit 4 C-Atomen, wie Methoxy, Halogen mit einer Atomnummer bis und mit 35, wie Fluor oder Chlor, Hydroxy und/oder Benzyloxy substituiertes Phenyl, wie 2,6-Dichlor-, 2,6-Difluor-, 2,6-Dimethyl-, 2-Chlor-6-methyl-, 4-Hydroxy-3-methoxy- oder 4-Benzyloxy-3-methoxy-phenyl, bedeutet, $Ar_2$ einen unsubstituierten oder durch Hydroxy substituierten Phenylen-, insbesondere 1,2-Phenylen-, oder Thienylen-, wie 3,4-Thienylen-rest, bedeutet, $R_1$ Wasserstoff oder 1-Phenylniederalkyl mit bis und mit 4 C-Atomen im Niederalkylteil, wie Benzyl, bedeutet, $R_2$ Niederalkyl mit bis und mit 4 C-Atomen, wie Methyl oder Isopropyl, bedeutet, $R_3$ Wasserstoff ist und Z Niederalkylen mit bis und mit 4 C-Atomen, wie Methylen oder Ethylen, oder Niederalkenylen mit 2 bis und mit 4 C-Atomen, wie Ethenylen, bedeutet, in freier Form oder in Form von Salzen.

Die Erfindung betrifft in besonderer Weise Verbindungen der Formel I, worin $Ar_1$ durch Niederalkyl mit bis und mit 4 C-Atomen, wie Methyl, Niederalkoxy mit bis und mit 4 C-Atomen, wie Methoxy, Halogen mit einer Atomnummer bis und mit 35, wie Fluor oder Chlor, Hydroxy und/oder Benzyloxy ein-, zwei-, drei- oder vierfach substituiertes Phenyl bedeu tet, $Ar_2$ unsubstituiertes 1,2-, 1,3- oder 1,4-Phenylen oder unsubstituiertes 3,4-Thienylen bedeutet, $R_1$ Wasserstoff, 1-Phenylniederalkyl mit bis und mit 4 C-Atomen im Niederalkylteil, wie Benzyl, oder $C_1$-$C_4$-Alkyl, wie Methyl, ist, $R_2$ $C_1$-$C_4$-Alkyl, wie Methyl, darstellt, $R_3$ Wasserstoff oder $C_2$-$C_5$-Alkanoyl, wie Acetyl oder Pivaloyl, ist und Z $C_1$-$C_4$-Alkylen, wie Methylen, Ethylen oder 1,3-Propylen, oder $C_2$-$C_4$-Alkenylen, wie Ethenylen, bedeutet, und ihre Salze.

Die Erfindung betrifft insbesondere Verbindungen der Formel I, worin $Ar_1$ durch Halogen mit einer Atomnummer bis und mit 35, wie Chlor, zweifach, insbesondere in den Positionen 2 und 6, substituiertes Phenyl bedeutet, $Ar_2$ 1,2-Phenylen bedeutet, $R_1$ Wasserstoff oder Benzyl bedeutet, $R_2$ Niederalkyl mit bis und mit 4 C-Atomen, wie Methyl, bedeutet, $R_3$ Wasserstoff ist und Z Methylen oder Ethylen bedeutet, in freier Form oder in Form von Salzen.

Die Erfindung betrifft in ganz besonderer Weise Verbindungen der Formel I, worin $Ar_1$ 2,6-Difluorphenyl oder 2,6-Dichlorphenyl ist, $Ar_2$ unsubstituiertes 1,2- oder 1,4-Phenylen oder unsubstituiertes 3,4-Thienylen darstellt, $R_1$ Wasserstoff oder Benzyl bedeutet, $R_2$ $C_1$-$C_4$-Alkyl, wie Methyl, ist, $R_3$ Wasserstoff ist und Z $C_1$-$C_4$-Alkylen, wie Methylen, Ethylen oder 1,3-Propylen, bedeutet, und ihre Salze.

Die Erfindung betrifft in allererster Linie Verbindungen der Formel I, worin $Ar_1$ 2,6-Dichlorphenyl ist, $Ar_2$ unsubstituiertes 1,2-Phenylen oder unsubstituiertes 3,4-Thienylen ist, $R_1$ Wasserstoff ist, $R_2$ $C_1$-$C_4$-Alkyl, wie Methyl, ist, $R_3$ Wasserstoff ist und Z Methylen bedeutet, und ihre Salze.

Die Erfindung betrifft namentlich die in den Beispielen genannten neuen Verbindungen der Formel I und ihre Salze.

Ein weiterer Gegenstand der Erfindung ist ein Verfahren zur Herstellung der Verbindungen der Formel I oder ihrer Salze in an sich bekannter Weise, z.B. dadurch gekennzeichnet, dass man

a) eine Verbindung der Formel

$$Ar_1 - \underset{R_1}{N} - Ar_2 - Z - COOH \qquad (IIa)$$

oder ein Salz und/oder ein reaktionsfähiges Derivat davon mit einer Verbindung der Formel

$R_3$- O - HN - $R_2$ (IIb)

oder einem Salz davon umsetzt oder

b) zur Herstellung einer Verbindung I, worin $R_3$ Wasserstoff ist, oder eines Salzes davon in einer Verbindung der Formel

$$Ar_1 - \underset{R_1}{N} - Ar_2 - Z - CO - \underset{R_2}{N} - OP_1 \qquad (III)$$

oder einem Salz davon die Schutzgruppe $P_1$ in Wasserstoff überführt oder

c) eine Verbindung der Formel

$$Ar_1 - \underset{R_1}{N} - H \qquad (IVa)$$

oder ein Salz davon mit einer Verbindung der Formel

$$X_1 - Ar_2 - Z - CO - \underset{\displaystyle R_2}{\underset{|}{N}} - O - R_3 \qquad (IVb),$$

worin $X_1$ reaktionsfähiges verestertes Hydroxy bedeutet, oder einem Salz davon umsetzt und jeweils gewünschtenfalls eine verfahrensgemäss oder auf andere Weise erhältliche Verbindung I in eine andere Verbindung I überführt, ein verfahrensgemäss erhältliches Isomerengemisch in die Komponenten auftrennt, ein verfahrensgemäss erhältliches Enantiomeren-beziehungsweise Diastereomerengemisch in die Enantiomeren beziehungsweise Diastereomeren aufspaltet und/oder eine verfahrensgemäss erhältliche freie Verbindung I in ein Salz oder ein verfahrensgemäss erhältliches Salz in die freie Verbindung I oder in ein anderes Salz umwandelt.

Die vor- und nachstehend in den Varianten beschriebenen Umsetzungen werden in an sich bekannter Weise durchgeführt, z.B. in Ab- oder üblicherweise in Anwesenheit eines geeigneten Lösungs- oder Verdünnungsmittels oder eines Gemisches derselben, wobei man je nach Bedarf unter Kühlen, bei Raumtemperatur oder unter Erwärmen, z.B. in einem Temperaturbereich von etwa -80 °C bis zur Siedetemperatur des Reaktionsmediums, vorzugsweise von etwa -10 °C bis etwa +100 °C, und, falls erforderlich, in einem geschlossenen Gefäss, unter Druck, in einer Inertgasatmosphäre und/oder unter wasserfreien Bedingungen arbeitet.

Das vor- und nachstehend aufgeführte Ausgangsmaterial, das für die Herstellung der Verbindungen I oder (ihrer Salze verwendet wird, ist zum Teil bekannt oder kann ebenfalls nach an sich bekannten Methoden, z.B. analog den vorstehend beschriebenen Verfahrensvarianten, hergestellt werden.

Für Salze von Verbindungen IIa, IIb, III, IVa und IVb gilt das vorstehend für Salze von Verbindungen I Gesagte in analoger Weise. Verbindungen IIa können zudem als Salze mit Ammoniak oder organischen Aminen, wie cyclischen Aminen, wie Mono-, Di- oder Triniederalkylaminen, wie Hydroxyniederalkylaminen, z.B. Mono-, Di- oder Trihydroxyniederalkylaminen, Hydroxyniederalkyl-niederalkyl-aminen oder Polyhydroxyniederalkylaminen, vorliegen. Cyclische Amine sind z.B. Morpholin, Thiomorpholin, Piperidin oder Pyrrolidin. Als Mononiederalkylamine kommen beispielsweise Ethyl-oder t-Butylamin, als Diniederalkylamine beispielsweise Diethyl- oder Diisopropylamin, als Triniederalkylamine beispielsweise Trimethyl- oder Triethylamin in Betracht. Entsprechende Hydroxyniederalkylamine sind z.B. Mono-, Di- oder Triethanolamin und Hydroxyniederalkyl-niederalkylamine sind z.B. N,N-Dimethylamino- oder N,N-Diethylamino-ethanol. Als Polyhydroxyniederalkylamin kommt z.B. Glucosamin in Frage.

Variante a):

Ein reaktionsfähiges Derivat einer Verbindung IIa ist beispielsweise ein reaktionsfähiges Carbonsäurederivat davon, z.B. ein entsprechender Ester, insbesondere ein aktivierter Ester, wie Niederalkylester, Cyanmethylester oder ein gegebenenfalls, z.B. durch Nitro, substituierter Phenylester, ein entsprechendes Amid, wie ein mit Ammoniak oder einem Amin, z.B. einem Niederalkyl- oder Diniederalkylamin, gebildetes Amid, ein entsprechendes Carbonsäureanhydrid, z.B. ein mit einer anorganischen Säure, wie einer Halogenwasserstoffsäure, oder mit einer Carbonsäure, wie einer Niederalkancarbonsäure, gebildetes Anhydrid, oder ein durch eines der nachstehend aufgeführten Kupplungsreagentien, gegebenenfalls in situ, gebildetes oder durch einen geeigneten Aktivierungskatalysator aktiviertes Carbonsäurederivat. Bevorzugte Ausgangsmaterialien sind die freie Säure IIa, der Cyanmethylester davon, der p-Nitrophenylester davon, ein Anhydrid davon mit einer Halogenwasserstoffsäure, z.B. das entsprechende Säurechlorid, sowie durch Kupplungsreagentien bzw. entsprechende Aktivierungskatalysatoren aktivierte Derivate, insbesondere der N-Succinimidester, davon.

Verbindungen IIb werden vorzugsweise in Salzform, z.B. als Hydrochlorid, eingesetzt.

Die Umsetzung mit einer Verbindung IIb oder einem Salz davon erfolgt z.B. in Gegenwart einer Base und gegebenenfalls eines Kupplungsreagens, dem ein geeigneter Aktivierungskatalysator zugesetzt sein kann. So erfolgt die Reaktion der freien Säure IIa mit einem Hydroxylamin IIb oder einem Salz davon vorteilhaft in Gegenwart eines Kupplungsreagens, insbesondere unter Zusatz eines Aktivierungskatalysators, und einer Base, während entsprechende reaktionsfähige Säurederivate vorteilhaft in Gegenwart einer Base umgesetzt werden. Bei ausreichender Basizität der Hydroxylaminkomponente erübrigt sich der Zusatz von Base.

Als Kupplungsreagentien können insbesondere Carbodiimide, z.B. N,N′-Di-$C_1$-$C_4$-alkyl- oder N,N′-Di-$C_5$-$C_7$-cycloalkyl-carbodiimide, wie N,N′-Di-isopropylcarbodiimid oder N,N′-Dicyclohexyl-carbodiimid, vorteilhaft unter Zusatz eines Aktivierungskatalysators, wie von N-Hydroxysuccinimid oder gegebenenfalls, z.B. durch Halogen, $C_1$-$C_7$-Alkyl oder $C_1$-$C_7$-Alkoxy, substituiertem N-Hydroxy-benzotriazol oder N-Hydroxy-5-norbornen-2,3-dicarboxamid, ferner $C_1$-$C_4$-Alkylhalogenoformiate, z.B. Isobutylchloroformiat, geeignete Carbonylverbindungen, z.B. N,N′-Carbonyldiimidazol, geeignete 1,2-Oxazoliumverbindungen, z.B. 2-Ethyl-5-phenyl-1,2-oxazolium-3′-sulfonat oder 2-tert-Butyl-5-methyl-isoxazolium-perchlorat, oder geeignete Acylaminoverbindungen, z.B. 2-Ethoxy-1-ethoxycarbonyl-1,2-dihydrochinolin, geeignete Phosphorylcyanamide bzw. -azide, z.B. Diethylphosphorylcyanamid oder Diphenylphosphorylazid, ferner Triphenylphosphan-disulfid oder 1-$C_1$-$C_4$-Alkyl-2-halogen-pyridinium-halogenide, z.B. 1-Methyl-2-chlor-pyridinium-iodid, eingesetzt werden.

Als Basen kommen beispielsweise Alkalimetallhydroxide, -hydride, -amide, -alkanolate, -carbonate, -triphenylmethylide, -di-$C_1$-$C_7$-alkylamide, -amino-$C_1$-$C_7$-alkylamide oder -$C_1$-$C_7$-alkylsilylamide, Naphthalinamine, $C_1$-$C_7$-Alkylamine, basische Heterocyclen, Ammoniumhydroxide sowie carbocyclische Amine in Frage. Beispielhaft seien Lithiumhydroxid, Natriumhydroxid, -hydrid, -amid, -ethylat, Kalium-tert-butanolat, -carbonat, Lithiumtriphenylmethylid, -diisopropylamid, Kalium-3-(aminopropyl)-amid, -bis-(trimethylsilyl)-amid, Dimethylaminonaphthalin, Di- oder Triethylamin, Pyridin, Benzyltrimethyl-ammoniumhydroxid, 1,5-Diaza-bicyclo[4.3.0]non-5-en (DBN) sowie 1,5-Diaza-bicyclo[5.4.0]undec-5-en (DBU) genannt.

Die in der Variante a) einzusetzenden Ausgangsmaterialien sind z.T. bekannt oder können in Analogie zu den bekannten Ausgangsmaterialien hergestellt werden.

Variante b):

Die Schutzgruppe $P_1$ in einer Verbindung III oder einem Salz davon ist eine der üblicherweise zum Schutz von OH-Gruppen verwendeten Schutzgruppen, vorzugsweise Benzyl oder Tetrahydropyranyl.

Die Ueberführung von $P_1$ in Wasserstoff, also die Abspaltung der Schutzgruppe, erfolgt in an sich bekannter Weise, beispielsweise durch Solvolyse oder Reduktion, z.B. katalytische Hydrogenolyse oder reduktive Spaltung mit Reduktionssystemen. So wird beispielsweise Benzyl durch katalytische Hydrogenolyse, z.B. unter Verwendung eines Edelmetallhydrierungskatalysators, wie Pd, Pd/C, Pt oder $PtO_2$, durch Solvolyse, z.B. mit HBr oder flüssigem HF, oder durch reduktive Spaltung, z.B. mit $Na/NH_3$, abgespalten, während Tetrahydropyranyl durch Solvolyse, z.B. mit einer Halogenwasserstoffsäure, wie Salzsäure, oder Trifluoressigsäure, entfernt wird.

Das Ausgangsmaterial für die Variante b) ist in an sich bekannter Weise zugänglich, beispielsweise unter Verwendung der unter Variante c) beschriebenen Methodik, wobei statt einer Verbindung IVb oder eines Salzes davon das entsprechende O-geschützte Derivat der Formel

$$X_1 - Ar_2 - Z - CO - \underset{\substack{| \\ R_2}}{N} - OP_1 \qquad \text{(IIIa)}$$

oder ein Salz davon eingesetzt wird.

Variante c):

Reaktionsfähiges verestertes Hydroxy $X_1$ in einer Verbindung IVb oder einem Salz davon ist insbesondere mit einer starken anorganischen Säure oder organischen Sulfonsäure verestertes Hydroxy, beispielsweise Halogen, wie Chlor, Brom oder Iod, Sulfonyloxy, wie Hydroxysulfonyloxy, Halogensulfonyloxy, z.B. Fluorsulfonyloxy, gegebenenfalls, z.B. durch Halogen, substituiertes ($C_1$-$C_4$-)Alkansulfonyloxy, z.B. Methan- oder Trifluormethansulfonyloxy, ($C_5$-$C_7$-)Cycloalkansulfonyloxy, z.B. Cyclohexansulfonyloxy, oder gegebenenfalls, z.B. durch ($C_1$-$C_4$-)Alkyl oder Halogen, substituiertes Benzolsulfonyloxy, z.B. p-Bromphenyl- oder p-Toluolsulfonyloxy. $X_1$ bedeutet vorzugsweise Halogen, in erster Linie Brom oder Iod, ferner Chlor.

Die Umsetzung von Verbindungen IVa oder ihren Salzen mit Verbindungen IVb oder ihren Salzen erfolgt in an sich bekannter Weise, z.B. in Gegenwart eines katalytischen Mittels und bei erhöhter Temperatur, z.B. in einem Temperaturbereich von etwa 50° C bis etwa 300° C, insbesondere zwischen etwa 100° C und etwa 200° C, wobei ein geeignetes hochsiedendes inertes Lösungsmittel, wie ein aromatischer Kohlenwasserstoff, z.B. Toluol oder ein Xylol, ein Niederalkanol, z.B. Amylalkohol, ein Sulfoxid, z.B. Trimethylsulfoxid, oder ein Amid, z.B. N,N-Dimethylformamid, Hexamethylphosphorsäuretriamid oder N-Methylpyrrolidon, verwendet wird.

Die Variante c) wird bevorzugt zur Herstellung von Verbindungen I, worin $Ar_2$ einen entsprechenden 1,3- oder 1,4-Phenylenrest bedeutet, oder deren Salzen angewendet.

Als katalytische Mittel kommen beispielsweise elementares Kupfer, in erster Linie in aktivierter Form

vorliegendes Kupfer, und/oder Kupfersalze, wie entsprechende Halogenide, wie Chloride, Bromide oder lodide, Sulfate oder Oxide, in Betracht. Bevorzugte Salze sind Kupfer(I)salze. Ferner kann vorteilhaft ein Alkalimetall- oder Erdalkalimetallhalogenid, wie Natrium- oder Kaliumiodid, zugesetzt werden. Ueblicherweise setzt man dem Reaktionsgemisch mindestens ein Aequivalent einer geeigneten Base, insbesondere eines Alkalimetallcarbonats, z.B. von Kaliumcarbonat, zu. Ferner eignet sich auch ein Amin, wie Pyridin.

Die Ausgangsstoffe für die Variante c) sind teilweise bekannt oder können in Analogie zu den bekannten Ausgangsstoffen hergestellt werden.

Eine verfahrensgemäss oder auf andere Weise erhältliche Verbindung I in freier Form oder in Salzform kann in an sich bekannter Weise in eine andere Verbindung I in freier Form oder in Salzform überführt werden.

Enthalten z.B. die Verbindungen I ungesättigte Gruppen, wie Niederalkenylen- oder Niederalkenylgruppen, können diese in an sich bekannter Weise in entsprechende gesättigte Reste überführt werden, beispielsweise durch katalytische Hydrierung in Gegenwart von Hydrierungskatalysatoren, wie Edelmetallen bzw. deren Derivaten, wie Oxiden, z.B. von Nickel, Raney-Nickel, Palladium oder Platinoxid, die gegebenenfalls auf Trägermaterialien, z.B. auf Kohle oder Calciumcarbonat, aufgezogen sein können. Die Hydrierung kann vorzugsweise bei Drucken zwischen etwa 1 und etwa 100 atm durchgeführt werden.

Wasserstoff $R_3$ kann gegen Niederalkanoylreste $R_3$ ausgetauscht werden. Die Niederalkanoylierung erfolgt in üblicher Weise, z.B. in Gegenwart eines geeigneten Niederalkanoylierungsreagens, in Anwesenheit eines geeigneten Lösungsmittels oder Lösungsmittelgemisches, unter schwachem Erwärmen oder bei Raumtemperatur, z.B. zwischen etwa 20° C und etwa 80° C, und gegebenenfalls in Gegenwart einer Base. Geeignete Niederalkanoylierungsreagentien sind z.B. entsprechende Niederalkanoylderivate, wie Niederalkanoylhalogenide, z.B. -bromide oder vorzugsweise -chloride, aktivierte Ester von Niederalkansäuren, z.B. Cyanmethylester, p-Nitrophenylester oder N-Succinimidester von Niederalkansäuren, oder vorzugsweise Niederalkansäureanhydride. Beispielhaft genannt seien Acetylchlorid, Pivaloylchlorid und Acetanhydrid. Geeignete Lösungsmittel sind z.B. inerte Lösungsmittel, wie Kohlenwasserstoffe, z.B. Benzol, Toluol oder Dichlormethan, oder dem Niederalkanoylrest des Niederalkanoylierungsreagens entsprechende Niederalkansäuren, z.B. Essigsäure im Falle von Acetanhydrid. Im Falle der Verwendung von Niederalkanoylhalogeniden ist vorteilhaft in Gegenwart einer Base zu arbeiten, z.B. in Gegenwart von Niederalkylaminen, wie Triethylamin, oder basischen Heterocyclen, wie Pyridin.

Umgekehrt kann Niederalkanoyl $R_3$ in Wasserstoff $R_3$ überführt werden, z.B. durch Solvolyse, wie milde Hydrolyse, z.B. Behandlung mit Wasser unter neutralen oder schwach sauren Bedingungen, z.B. Einwirkung von verdünntwässrigen Mineral- oder Carbonsäuren, wie von verdünnter Salz- oder Essigsäure.

Gegebenenfalls substituierte Phenylniederalkoxysubstituenten im Ring $Ar_1$ können in üblicher Weise in Hydroxysubstituenten überführt werden, z.B. durch Solvolyse oder Reduktion, beispielsweise analog der Methode, die unter der Variante b) für die Abspaltung von Benzylschutzgruppen $P_1$ beschrieben wird. In analoger Weise lassen sich auch Phenylniederalkylreste $R_1$ gegen Wasserstoff $R_1$ austauschen.

In Aminogruppen $-NR_1-$ können in üblicher Weise aliphatische Reste $R_1$ gegen Wasserstoff $R_1$ und Wasserstoff $R_1$ gegen aliphatische Reste $R_1$ und gegen Phenylniederalkyl $R_1$ ausgetauscht werden.

In Gruppen $-O-R_3$ können in üblicher Weise Niederalkylreste $R_3$ gegen Wasserstoff $R_3$ und Wasserstoff $R_3$ gegen Niederalkyl $R_3$ ausgetauscht werden.

Salze von Verbindungen I können in an sich bekannter Weise hergestellt werden. So erhält man beispielsweise Säureadditionssalze von Verbindungen I durch Behandeln mit einer geeigneten Säure oder einem geeigneten Ionenaustauscherreagens. Salze mit Basen erhält man z.B. durch Behandeln mit einer geeigneten Base oder einem geeigneten Ionenaustauscherreagens. Salze von Verbindungen I können in üblicher Weise in die freien Verbindungen I überführt werden, Säureadditionssalze z.B. durch Behandeln mit einem geeigneten basischen Mittel oder einem geeigneten Ionenaustauscherreagens, Salze mit Basen z.B. durch Behandeln mit einer geeigneten starken Säure oder einem geeigneten Ionenaustauscherreagens.

Salze von Verbindungen I können in an sich bekannter Weise in andere Salze von Verbindungen I umgewandelt werden, Säureadditionssalze beispielsweise in andere Säureadditionssalze, z.B. durch Behandeln eines Salzes einer anorganischen Säure, wie eines Hydrochlorids, mit einem geeigneten Metallsalz, wie einem Natrium-, Barium- oder Silbersalz, einer Säure, z.B. mit Silberacetat, in einem geeigneten Lösungsmittel, in welchem ein sich bildendes anorganisches Salz, z.B. Silberchlorid, unlöslich ist und damit aus dem Reaktionsgemisch ausscheidet.

Je nach Verfahrensweise bzw. Reaktionsbedingungen können die Verbindungen I mit salzbildenden, insbesondere basischen, Eigenschaften in freier Form oder in Form von Salzen erhalten werden.

Infolge der engen Beziehung zwischen der Verbindung I in freier Form und in Form ihrer Salze sind im vorausgegangenen und nachfolgend unter der freien Verbindung I bzw. ihren Salzen sinn- und zweckgemäss gegebenenfalls auch die entsprechenden Salze bzw. die freie Verbindung I zu verstehen.

Die Verbindungen I einschliesslich ihrer Salze von salzbildenden Verbindungen können auch in Form ihrer Hydrate erhalten werden und/oder andere, beispielsweise gegebenenfalls zur Kristallisation von in fester Form vorliegenden Verbindungen verwendete, Lösungsmittel einschliessen.

Die Verbindungen I und ihre Salze können, je nach Wahl der Ausgangsstoffe und Arbeitsweisen, in Form eines der möglichen Isomeren oder als Gemisch derselben vorliegen. Dabei sind, in Abhängigkeit von der Molekülsymmetrie, z.B. je nach Anzahl, absoluter und relativer Konfiguration der Chiralitätszentren, wie asymmetrischen C-Atome, als reine Isomere z.B. reine Enantiomere und/oder reine Diastereomere, wie reine cis/trans-Isomere oder meso-Verbindungen, erhältlich. Entsprechend können als Isomerengemische z.B. Enantiomerengemische, wie Racemate, Diastereomerengemische oder Racematgemische vorliegen. Asymmetrische C-Atome können in den Verbindungen I und ihren Salzen z.B. in entsprechenden Niederalkylresten $R_1$, $R_2$ und $R_3$ und Niederalkylenresten Z auftreten.

Erhaltene Diastereomerengemische und Racematgemische können aufgrund der physikalisch-chemischen Unterschiede der Bestandteile in bekannter Weise in die reinen Diastereomeren oder Racemate aufgetrennt werden, beispielsweise durch fraktionierte Kristallisation, Destillation und/oder Chromatographie.

Erhaltene Enantiomerengemische, wie Racemate, lassen sich nach bekannten Methoden in die Enantiomeren zerlegen, beispielsweise durch Umkristallisation aus einem optisch aktiven Lösungsmittel, Chromatographie an chiralen Adsorbentien, mit Hilfe von geeigneten Mikroorganismen, durch Spaltung mit spezifischen, immobilisierten Enzymen, über die Bildung von Einschlussverbindungen, z.B. unter Verwendung chiraler Kronenether, wobei nur ein Enantiomeres komplexiert wird, oder durch Ueberführung in diastereomere Salze, z.B. durch Umsetzung eines basischen Endstoffracemats mit einer optisch aktiven Säure, wie Carbonsäure, z.B. Wein-oder Aepfelsäure, oder Sulfonsäure, z.B. Camphersulfonsäure, und Trennung des auf diese Weise erhaltenen Diastereomerengemisches, z.B. auf Grund ihrer verschiedenen Löslichkeiten, in die Diastereomeren, aus denen das gewünschte Enantiomere durch Einwirkung geeigneter Mittel freigesetzt werden kann. Vorteilhaft isoliert man das wirksamere Enantiomere.

Die Erfindung betrifft auch diejenigen Ausführungsformen des Verfahrens, nach denen man von einer auf irgendeiner Stufe des Verfahrens als Zwischenprodukt erhältlichen Verbindung ausgeht und die fehlenden Schritte durchführt oder einen Ausgangsstoff in Form eines Derivates bzw. Salzes und/oder seiner Racemate bzw. Enantiomeren verwendet oder insbesondere unter den Reaktionsbedingungen bildet.

Beim Verfahren der vorliegenden Erfindung werden vorzugsweise solche Ausgangsstoffe und Zwischenprodukte, jeweils in freier Form oder in Salzform, verwendet, welche zu den eingangs als besonders wertvoll geschilderten Verbindungen I bzw. deren Salzen führen. Neue Ausgangsstoffe und Zwischenprodukte, jeweils in freier Form oder in Salzform, für die Herstellung der Verbindungen I bzw. ihrer Salze, ihre Verwendung und Verfahren zu ihrer Herstellung bilden ebenfalls einen Gegenstand der Erfindung, wobei die Variablen $R_1$, $R_2$, $R_3$, $Ar_1$, $Ar_2$ und Z die für die Verbindungen I angegebenen Bedeutungen haben.

Die Erfindung betrifft ebenfalls die Verwendung der Verbindungen I und ihrer pharmazeutisch verwendbaren Salze zur Behandlung entzündlicher, allergischer, asthmatischer und anaphylaktischer Symptome, vorzugsweise in Form von pharmazeutisch verwendbaren Zubereitungen, insbesondere in einem Verfahren zur therapeutischen Behandlung des tierischen oder menschlichen Körpers.

Die Erfindung betrifft gleichfalls pharmazeutische Präparate, die eine Verbindung I oder ein pharmazeutisch verwendbares Salz davon als Wirkstoff enthalten, sowie Verfahren zu ihrer Herstellung. Bei diesen pharmazeutischen Präparaten handelt es sich um solche zur enteralen, wie oralen, ferner rektalen, oder topischen oder parenteralen Verabreichung an Warmblüter, wobei der pharmakologische Wirkstoff allein oder zusammen mit einem pharmazeutisch anwendbaren Trägermaterial enthalten ist. Die pharmazeutischen Präparate enthalten z.B. von etwa 0,1 % bis 100 %, vorzugsweise von etwa 1 % bis etwa 50 %, des Wirkstoffs. Pharmazeutische Präparate zur enteralen bzw. parenteralen Verabreichung sind z.B. solche in Dosiseinheitsformen, wie Dragées, Tabletten, Kapseln oder Suppositorien, ferner Ampullen. Diese werden in an sich bekannter Weise, z.B. mittels konventioneller Misch-, Granulier-, Dragier-, Lösungs- oder Lyophilisierungsverfahren, hergestellt. So kann man pharmazeutische Präparate zur oralen Anwendung erhalten, indem man den Wirkstoff mit festen Trägerstoffen kombiniert, ein erhaltenes Gemisch gegebenenfalls granuliert und das Gemisch bzw. Granulat, wenn erwünscht oder notwendig nach Zugabe von geeigneten Hilfsstoffen, zu Tabletten oder Dragée-Kernen verarbeitet.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärkekleister, unter Verwendung z.B. von Mais-, Weizen-, Reis- oder Kartoffelstärke, Gelatine, Tragakanth, Methylcellulose und/oder Polyvinylpyrrolidon, wenn erwünscht, Sprengmittel, wie die obengenannten Stärken, ferner Carboxymethylstärke, quervernetzes Polyvinylpyrrolidon, Agar, Alginsäure oder ein Salz davon, wie Natriumalginat, Hilfsmittel sind in erster Linie Fliess-, Fliessregulier- und Schmiermittel, z.B. Kieselsäure, Talk, Stearinsäure oder Salze davon, wie Magnesium-oder Calciumstearat, und/oder Polyethylenglykol. Dragée-Kerne werden mit geeigneten, gegebenenfalls Magensaft-resistenten Ueberzügen versehen, wobei man u.a. konzentrierte Zuckerlösungen, welche gegebenenfalls arabischen Gummi, Talk, Polyvinylpyrrolidon, Polyethylenglykol und/oder Titandioxid enthalten, Lacklösungen in geeigneten organischen Lösungsmitteln oder Lösungsmittelgemische oder, zur Herstellung von Magensaft-resistenten Ueberzügen, Lösungen von geeigneten Cellulosepräparaten, wie Acetylcellulosephthalat oder Hydroxypropylmethylcellulosephthalat, verwendet. Den Tabletten oder Dragée-Ueberzügen können Farbstoffe oder Pigmente, z.B. zur Identifizierung oder zur Kennzeichnung verschiedener Wirkstoffdosen, beigefügt werden.

Weitere oral anwendbare pharmazeutische Präparate sind Steckkapseln aus Gelatine, sowie weiche, geschlossene Kapseln aus Gelatine und einem Weichmacher, wie Glycerin oder Sorbitol. Die Steckkapseln können den Wirkstoff in Form eines Granulats, z.B. im Gemisch mit Füllstoffen, wie Lactose, Bindemitteln, wie Stärken, und/oder Gleitmitteln, wie Talk oder Magnesiumstearat, und gegebenenfalls Stabilisatoren, enthalten. In weichen Kapseln ist der Wirkstoff vorzugsweise in geeigneten Flüssigkeiten, wie fetten Oelen, Paraffinöl oder flüssigen Polyethylenglykolen, gelöst oder suspendiert, wobei ebenfalls Stabilisatoren zugefügt sein können.

Als rektal anwendbare pharmazeutische Präparate kommen z.B. Suppositorien in Betracht, welche aus einer Kombination des Wirkstoffs mit einer Suppositoriengrundmasse bestehen. Als Suppositorienmasse eignen sich z.B. natürliche oder synthetische Triglyceride, Paraffinkohlenwasserstoffe, Polyethylenglykole oder höhere Alkanole. Ferner können auch Gelatine-Rektalkapseln verwendet werden, die eine Kombination des Wirkstoffs mit einem Grundmassenstoff enthalten. Als Grundmassenstoffe kommen z.B. flüssige Triglyceride, Polyethylenglykole oder Paraffinkohlenwasserstoffe in Frage.

Als topisch anwendbare pharmazeutische Präparate kommen in erster Linie Cremes, Salben, Schäume, Tinkturen und Lösungen in Frage, die von etwa 0,5 bis etwa 20 % des Wirkstoffs enthalten.

Cremes sind Oel-in-Wasser Emulsionen, die mehr als 50 % Wasser aufweisen. Als ölige Grundlage verwendet man in erster Linie Fettalkohole, z.B. Lauryl-, Cetyl- oder Stearylalkohol, Fettsäuren, z.B. Palmitin- oder Stearinsäure, flüssige bis feste Wachse, z.B. Isopropylmyristat, Wollwachs oder Bienenwachs, und/oder Kohlenwasserstoffe, z.B. Vaseline (Petrolatum) oder Paraffinöl. Als Emulgatoren kommen oberflächenaktive Substanzen mit vorwiegend hydrophilen Eigenschaften in Frage, wie entsprechende nichtionische Emulgatoren, z.B. Fettsäureester von Polyalkoholen oder Ethylenoxidaddukte davon, wie Polyglycerinfettsäureester oder Polyoxyethylensorbitanfettsäureester (Tweens), ferner Polyoxyethylenfettalkoholether oder -fettsäureester, oder entsprechende ionische Emulgatoren, wie Alkalimetallsalze von Fettalkoholsulfaten, z.B. Natriumlaurylsulfat, Natriumcetylsulfat oder Natriumstearylsulfat, die man üblicherweise in Gegenwart von Fettalkoholen, z.B. Cetylalkohol oder Stearylalkohol, verwendet. Zusätze zur Wasserphase sind u.a. Mittel, welche die Austrocknung der Creme vermindern, z.B. Polyalkohole, wie Glycerin, Sorbit, Propylenglykol und/oder Polyethylenglykole, ferner Konservierungsmittel, Riechstoffe, etc..

Salben sind Wasser-in-Oel-Emulsionen, die bis zu 70 %, vorzugsweise jedoch von etwa 20 % bis etwa 50 %, Wasser oder wässrige Phasen enthalten. Als Fettphase kommen in erster Linie Kohlenwasserstoffe, z.B. Vaseline, Paraffinöl und/oder Hartparaffine in Frage, die zur Verbesserung des Wasserbindungsvermögens vorzugsweise geeignete Hydroxyverbindungen, wie Fettalkohole oder Ester davon, z.B. Cetylalkohol oder Wollwachsalkohole, bzw. Wollwachs enthalten. Emulgatoren sind entsprechende lipophile Substanzen, wie Sorbitanfettsäureester (Spans), z.B. Sorbitanoleat und/oder Sorbitanisostearat. Zusätze zur Wasserphase sind u.a. Feuchthaltungsmittel, wie Polyalkohole, z.B. Glycerin, Propylenglykol, Sorbit und/oder Polyethylenglykol, sowie Konservierungsmittel, Riechstoffe, etc..

Fettsalben sind wasserfrei und enthalten als Grundlage insbesondere Kohlenwasserstoff, z.B. Paraffin, Vaseline und/oder flüssige Paraffine, ferner natürliches oder partialsynthetisches Fett, z.B. Kokosfettsäuretriglycerid, oder vorzugsweise gehärtete Oele, z.B. hydriertes Erdnuss- oder Rizinusöl, ferner Fettsäurepartialester des Glycerins, z.B. Glycerinmono- und -distearat, sowie z.B. die im Zusammenhang mit den Salben erwähnten, die Wasseraufnahmefähigkeit steigernden Fettalkohole, Emulgatoren und/oder Zusätze.

Pasten sind Cremes und Salben mit sekretabsorbierenden Puderbestandteilen, wie Metalloxiden, z.B. Titanoxid oder Zinkoxid, ferner Talk und/oder Aluminiumsilikaten, welche die Aufgabe haben, vorhandene Feuchtigkeit oder Sekrete zu binden.

Schäume werden aus Druckbehältern verabreicht und sind in Aerosolform vorliegende flüssige Oel-in-Wasser-Emulsionen, wobei halogenierte Kohlenwasserstoffe, wie Chlorfluorniederalkane, z.B. Dichlordifluormethan und Dichlortetrafluorethan, als Treibmittel verwendet werden. Als Oelphase verwendet man u.a. Kohlenwasserstoffe, z.B. Paraffinöl, Fettalkohole, z.B. Cetylalkohol, Fettsäureester, z.B. Isopropylmyristat, und/oder andere Wachse. Als Emulgatoren verwendet man u.a. Gemische von solchen mit vorwiegend hydrophilen Eigenschaften, wie Polyoxyethylensorbitan-fettsäureester (Tweens), und solchen mit vorwiegend lipophilen Eigenschaften, wie Sorbitanfettsäureester (Spans). Dazu kommen die üblichen Zusätze, wie

Konservierungsmittel, etc.

Tinkturen und Lösungen weisen meistens eine wässerigäthanolische Grundlage auf, der u.a. Polyalkohole, z.B. Glycerin, Glykole, und/oder Polyethylenglykol, als Feuchthaltemittel zur Herabsetzung der Verdunstung, und rückfettende Substanzen, wie Fettsäureester mit niedrigen Polyethylenglykolen, d.h. im wässrigen Gemisch lösliche, lipophile Substanzen als Ersatz für die der Haut mit dem Aethanol entzogenen Fettsubstanzen, und, falls notwendig, andere Hilfs- und Zusatzmittel beigegeben sind.

Die Herstellung der topisch verwendbaren pharmazeutischen Präparate erfolgt in an sich bekannter Weise, z.B. durch Lösen oder Suspendieren des Wirkstoffs in der Grundlage oder in einem Teil davon, falls notwendig. Bei Verarbeitung des Wirkstoffs als Lösung wird dieser in der Regel von der Emulgierung in einer der beiden Phasen gelöst; bei Verarbeitung als Suspension wird er nach der Emulgierung mit einem Teil der Grundlage vermischt und dann dem Rest der Formulierung beigegeben.

Zur parenteralen Verabreichung eignen sich in erster Linie wässrige Lösungen eines Wirkstoffs in wasserlöslicher Form, ferner Suspensionen des Wirkstoffs, wie entsprechende ölige Injektionssuspensionen, wobei man geeignete lipophile Lösungsmittel oder Vehikel, wie fette Oele, z.B. Sesamöl, oder synthetische Fettsäureester, z.B. Ethyloleat oder Triglyceride, verwendet, oder wässrige Injektionssuspensionen, welche viskositätserhöhende Stoffe, z.B. Natrium-carboxymethylcellulose, Sorbit und/oder Dextran, und gegebenenfalls auch Stabilisatoren enthalten.

Die Dosierung des Wirkstoffs hängt von verschiedenen Faktoren, wie der Warmblüter-Spezies, dem Alter und dem individuellen Zustand sowie der Applikationsweise, ab. Im Normalfall ist für einen etwa 75 kg schweren Warmblüter bei oraler Applikation eine ungefähre Tagesdosis von etwa 20 mg bis etwa 3000 mg, insbesondere von etwa 150 mg bis etwa 1500 mg, vorteilhaft in mehreren gleichen Teildosen, zu veranschlagen.

Die nachfolgenden Beispiele illustrieren die oben beschriebene Erfindung; sie sollen jedoch diese in ihrem Umfang in keiner Weise einschränken. Temperaturen sind in Celsiusgraden angegeben.

Beispiel 1:

Zu einer Lösung von 14,8 g o-[(2,6-Dichlorphenyl)-amino]-phenylessigsäure (beschrieben in GB 1,132,318) in 100 ml abs. Tetrahydrofuran setzt man bei Raumtemperatur 8,92 g 1,1'-Carbonyl-diimidazol und anschliessend 8,35 g N-Methyl-hydroxylamin-hydrochlorid und 17 ml Ethyl-diisopropylamin zu. Die Mischung wird eine Stunde bei 40° gerührt, wobei tropfenweise 1 ml Ethyl-diisopropylamin zugesetzt wird. Man kühlt ab und engt die Mischung unter 11 Torr bei 40° zur Trockne ein. Den Rückstand schüttelt man mit 300 ml Ethylacetat und 50 ml 2-n Salzsäure. Die organische Phase wird abgetrennt und dreimal mit je 30 ml 2-n Natriumcarbonatlösung und 50 ml Wasser extrahiert, abgetrennt, über Magnesiumsulfat getrocknet und unter 11 Torr eingedampft. Den Rückstand kristallisiert man aus Ether-Petrolether. Die [o-[(2,6-Dichlorphenyl)-amino]-phenyl]-aceto-N-methyl-hydroxamsäure schmilzt bei 135-137°.

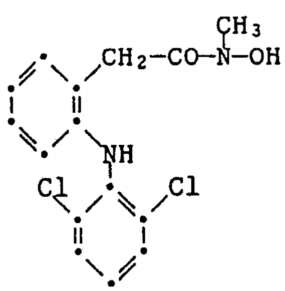

Beispiel 2:

In analoger Weise wie in Beispiel 1 beschrieben kann man die [o-[(2,6-Dichlor-4-benzyloxy-3-methoxyphenyl)-amino]-phenyl]-aceto-N-methyl-hydroxamsäure, Smp. 152-154° (aus Ethylacetat-Petrolether), ausgehend von o-[(2,6-Dichlor-4-benzyloxy-3-methoxyphenyl)-amino]-phenylessigsäure, Smp. 166-168° (aus Ether-Petrolether), herstellen.

Das Ausgangsmaterial kann z.B. wie folgt hergestellt werden:

a) Eine Lösung von 16,5 g 2,6-Dichlor-3-methoxyanilin (US Pat. 3 238 201, Parke-Davis) in 5,2 g

Eisessig wird unter Rühren tropfenweise mit 6,8 g Acetylchlorid versetzt, wobei sich ein Kristallbrei bildet. Die Suspension wird anschliessend 30 Minuten bei 45° gerührt, abgekühlt und auf 200 g Eis gegossen. Die Suspension wird mit 300 ml Methylenchlorid geschüttelt. Man trennt die organische Phase ab und extrahiert sie zweimal mit 50 ml Wasser, zweimal mit 2N-Kaliumbicarbonatlösung und nochmals mit Wasser, trocknet sie über Magnesiumsulfat und engt unter 11 Torr bei 40° zur Trockne ein. Den Rückstand, das N-Acetyl-2,6-dichlor-3-methoxyanilin, kristallisiert man aus Methanol-Wasser, Smp. 163-164°.

b) Ein Gemisch aus 175 ml conc. Schwefelsäure und 175 ml Salpetersäure (65 %) wird auf -40° abgekühlt und portionenweise unter Rühren mit 65,0 g N-Acetyl-2,6-dichlor-3-methoxyanilin versetzt. Die Mischung wird zwei Stunden bei -10° gerührt und anschliessend auf 2000 ml Eiswasser gegossen. Die ausgeschiedenen Kristalle werden abfiltriert, mit 3000 ml Eiswasser gewaschen und in 1500 ml Ethylacetat gelöst. Die organische Phase wird mit 200 ml Wasser gewaschen, über Magnesium sulfat getrocknet und unter 11 Torr bei 50° eingedampft. Den Rückstand kristallisiert man aus Ethylenacetat-Ether. Das N-Acetyl-2,6-dichlor-3-methoxy-4-nitroanilin schmilzt bei 175-176°.

c) 133,0 g N-Acetyl-2,6-dichlor-3-methoxy-4-nitroanilin, gelöst in 1400 ml Tetrahydrofuran, werden nach Zusatz von 14,0 g Raney-Nickel bei Raumtemperatur hydriert. Nach 27 Stunden wird vom Katalysator abfiltriert und das Filtrat unter 11 Torr bei 40° eingedampft. Den Rückstand kristallisiert man aus Methylenchlorid-Petrolether. Das 1-Acetamido-4-amino-2,6-dichlor-3-methoxybenzol schmilzt bei 128-131°.

d) Zu 44,8 g Bortrifluorethyletherat wird unter Rühren bei -20° eine Lösung von 52,6 g 1-Acetamido-4-amino-2,6-dichlor-3-methoxybenzol in 630 ml Tetrahydrofuran während 45 Minuten zugetropft. Die Mischung wird während 30 Minuten bei -15° gerührt und mit einer Lösung von 26,0 g tert.-Butylnitrit in 215 ml Tetrahydrofuran tropfenweise versetzt. Man rührt anschliessend 30 Minuten bei -15°, wobei sich orange Kristalle abscheiden. Das Reaktionsgemisch wird 45 Minuten bei 0° gerührt und mit 200 ml Hexan verdünnt. Die Kristalle, das 4-Acetamido-3,5-dichlor-2-methoxy-benzoldiazoniumtetrafluorborat, werden sofort weiterumgesetzt. Eine Mischung von 20,0 g Diazoniumsalz, 1500 ml Wasser und 500 ml Ethylacetat wird bei 15° unter Rühren während 90 Minuten mit einer Quarzlampe 1000 Watt beleuchtet. Die organische Phase wird abgetrennt, mit 100 ml Wasser gewaschen, über Magnesiumsulfat getrocknet und unter 11 Torr bei 40° eingeengt. Den Rückstand chromatographiert man an 150 g Silicagel. Die Fraktionen 1-12, eluiert mit je 800 ml Chloroform-Methanol (99:1), werden verworfen. Die Fraktionen 13-20, eluiert mit je 800 ml Chloroform-Methanol (98:2), werden vereinigt und unter 11 Torr bei 40° eingedampft. Den Rückstand kristallisiert man aus Ether-Petrolether. Das N-Acetyl-2,6-dichlor-3-methoxy-4-hydroxyanilin schmilzt bei 174-176°.

e) Eine Mischung von 26,0 g N-Acetyl-2,6-dichlor-3-methoxy-4-hydroxyanilin, 16,8 g Kaliumcarbonat und 590 ml Dimethylformamid wird drei Stunden bei 100° erhitzt, mit 13,9 ml Benzylchlorid versetzt und weitere 40 Minuten bei 100° gerührt. Man dampft unter 11 Torr bei 80° ein. Den Rückstand schüttelt man mit 200 ml Chloroform und 100 ml Wasser. Die organische Phase wird abgetrennt, über Magnesiumsulfat getrocknet und unter 11 Torr bei 40° zur Trockene eingedampft. Den Rückstand chromatographiert man an 400 g Silicagel. Die Fraktionen 1-6, eluiert mit je 700 ml Chloroform, werden vereinigt und unter 11 Torr bei 40° zur Trockne eingedampft. Den Rückstand kristallisiert man aus Chloroform-Ether. Das N-Acetyl-2,6-dichlor-4-benzyloxy-3-methoxyanilin schmilzt bei 162-164°.

f) Eine Mischung von 15,0 g N-Acetyl-2,6-dichlor-4-benzyloxy-3-methoxyanilin, 60,0 g Kaliumhydroxid, 300 ml Ethanol und 10 ml Wasser wird 48 Stunden unter Rückfluss erhitzt. Man destilliert das Ethanol unter 11 Torr bei 40° ab und versetzt den Rückstand mit 100 ml Eiswasser. Dann wird dreimal mt je 200 ml Ether extrahiert. Die vereinigten Etherphasen werden mit 100 ml Wasser gewaschen, über Magnesiumsulfat getrocknet und unter 11 Torr bei 40° eingedampft. Den Rückstand chromatograhiert man an 380 g Silicagel. Die Fraktionen 1-4, eluiert mit je 400 ml Chloroform, werden vereinigt und unter 11 Torr bei 40° eingedampft. Den Rückstand kristallisiert man aus Ether-Petrolether. Das 2,6-Dichlor-4-benzyloxy-3-methoxyanilin schmilzt bei 43-45°.

g) Eine Mischung aus 4,35 g N,N-Dimethyl-o-iodophenylacetamid [GB-2 027 028; C.A. 94, 15402x (1981)], 8,7 g 2,6-Dichlor-4-benzyloxy-3-methoxyanilin, 1,58 g wasserfreiem Kaliumcarbonat, 0,5 g aktiviertem (Org. Synth. 1964, 3, 339) Kupferpulver, 0,2 g Kupfer-I-iodid und 60 ml Toluol wird während 38 Stunden unter Rühren zum Rückfluss erhitzt, während das gebildete Wasser in einem mit Molekularsieb Typ 4 Å gefüllten Wasserabscheider abgetrennt wird. Die Mischung wird heiss filtriert. Man engt das Filtrat unter 11 Torr bei 60° zur Trockne ein. Der Rückstand wird an 200 g Silicagel chromatographiert. Die Fraktionen 1-7, eluiert mit je 400 ml Toluol-Ethylacetat (90:10) werden verworfen. Die Fraktionen 8-15, eluiert mit je 400 ml Toluol-Ethylacetat (80:20), werden vereinigt und unter 11 Torr bei 60° zur Trockne eingedampft. Der Rückstand, ein gelbes Oel, wird aus Ether-Petrolether kristallisiert. Das N,N-Dimethyl-o-[(2,6-dichlor-4-benzyloxy-3-methoxyphenyl)-amino]-phenylacetamid schmilzt bei 104-105°.

h) Eine Mischung von 1,2 g N,N-Dimethyl-o-[(2,6-dichlor-4-benzyloxy-3-methoxyphenyl)-amino]-phenylacetamid, 4,0 g KOH in 60 ml Ethanol und 6,0 ml Wasser wird vier Stunden unter Rückfluss erhitzt. Das Lösungsmittel wird unter 11 Torr bei 40° abdestilliert, der Rückstand mit 50 ml Eiswasser versetzt und die Mischung bei 0° mit 2N-Salzsäure angesäuert. Man extrahiert dreimal mit je 70 ml Ether. Die vereinigten Etherphasen werden zweimal mit je 50 ml Wasser gewaschen, über Magnesiumsulfat getrocknet und unter 11 Torr bei 40° zur Trockne eingedampft. Der Rückstand wird an 20 g Silicagel chromatographiert. Die Fraktionen 1-3, eluiert mit je 100 ml Chloroform-Methanol (97:3), werden verworfen. Die Fraktionen 4-10, eluiert mit dem gleichen Gemisch, werden vereinigt und unter 11 Torr bei 40° zur Trockne eingedampft. Den Rückstand kristallisiert man aus Ether-Petrolether. Die o-[(2,6-Dichlor-4-benzyloxy-3-methoxyphenyl)-amino]-phenylessigsäure schmilzt bei 166-168°.

Beispiel 3:

In analoger Weise wie in Beispiel 1 beschrieben kann man herstellen:
[o-[(2,6-Dichlor-4-hydroxy-3-methoxy-phenyl)-amino]-phenyl]-aceto-N-methyl-hydroxamsäure, Smp. 164-166° (aus Ether-Petrolether), ausgehend von o-[(2,6-Dichlor-4-hydroxy-3-methoxyphenyl)-amino]-phenylessigsäure, Smp. 164-166° (aus Ether-Petrolether);
[o-[(2,6-Dichlorphenyl)-N-benzyl-amino]-phenyl]-aceto-N-methyl-hydroxamsäure, Smp. 144-147° (aus Ether-Petrolether), ausgehend von o-[(2,6-Dichlorphenyl)-N-benzyl-amino]-phenylessigsäure, Smp. 172-174° (aus Ether-Petrolether), beschrieben von: Fukushima Sh. et al. (Nissan Chemical Ind. Ltd.) Jpn. Kokai Tokyo Koho JP 54/160336; Chem. Abstr. 93(9): 94963m.
[o-(2,6-Dichlorphenyl)-amino]-cinnamo-N-methyl-hydroxamsäure, Smp. 186-188° (aus Ether-Petrolether), ausgehend von o-[(2,6-Dichlorphenyl)-amino]-zimtsäure, Smp. 222-224° (aus Ethanol); und
[o-[(2,6-Difluorphenyl)-amino]-phenyl]-aceto-N-methyl-hydroxamsäure, Smp. 145-147° (aus Ether-Petrolether), ausgehend von o-[(2,6-Difluorphenyl)-amino]-phenylessigsäure, Smp. 155-159° (aus Ether-Petrolether.

Beispiel 4:

1,48 g [o-[(2,6-Dichlor-4-benzyloxy-3-methoxy-phenyl)-amino]-phenyl]-aceto-N-methyl-hydroxamsäure werden in 30 ml Ethylacetat gelöst und nach Zusatz von 0,3 g Palladium-Kohle (5 %) bei Raumtemperatur hydriert. Nach 1,5 Stunden kommt die Hydrierung zum Stillstand. Man filtriert vom Katalysator ab und engt das Filtrat unter 11 Torr bei 40° zur Trockne ein. Den Rückstand löst man in wenig Ether. Nach Zusatz von Petrolether kristallisiert die [o-[(2,6-Dichlor-4-hydroxy-3-methoxy-phenyl)-amino]-phenyl]-aceto-N-methyl-hydroxamsäure aus Smp. 164-166°.

Beispiel 5:

In analoger Weise wie in Beispiel 1 beschrieben kann man die 3-[o-[(2,6-Dichlorphenyl)-amino]-phenyl]-propio-N-methyl-hydroxamsäure, Smp. 167-170° (aus Ethylacetat-Petrolether), ausgehend von 3-[o-[(2,6-Dichlorphenyl)-amino]-phenyl]propionsäure herstellen.

Das Ausgangsmaterial kann z.B. wie folgt hergestellt werden:
Eine Lösung von 18,5 g o-[(2,6-Dichlorphenyl)-amino]-zimtsäure (beschrieben in FR Patent 1,555,647) in 400 ml Methanol und 65 ml 1-normale Natronlauge wird nach Zusatz von 5,0 g Platin-Kohle (5 %) während 60 Stunden bei Raumtemperatur hydriert. Man filtriert vom Katalysator ab und engt unter 11 Torr bei 60° zur Trockne ein. Den Rückstand löst man in 700 ml Wasser. Die wässrige Phase wird mit 200 ml Ethylacetat extrahiert und anschliessend mit 2-normaler Salzsäure angesäuert. Das ausgeschiedene Oel wird zweimal mit je 300 ml Ethylacetat extrahiert. Die vereinigten organischen Phasen werden mit 100 ml Wasser gewaschen, über Magnesiumsulfat getrocknet und unter 11 Torr bei 40° eingeengt. Den Rückstand kristallisiert man zweimal aus Ethylacetat-Petrolether. Die 3-[o-[(2,6-Dichlorphenyl)-amino]-phenyl]-propionsäure schmilzt bei 123-125°.

Beispiel 6:

In analoger Weise wie in Beispiel 1 beschrieben kann man die 4-[o-[(2,6-Dichlorphenyl)-amino]-phenyl]-butyro-N-methyl-hydroxamsäure, Smp. 132-134° (aus Ether-Petrolether), herstellen.

Das Ausgangsmaterial kann z.B. wie folgt hergestellt werden:

a) Eine Lösung von 19,95 g o-[(2,6-Dichlorphenyl)-amino]-benzaldehyd (A. Sallmann, R. Pfister, Geigy AG, Schweiz. Pat. 475 200) in 150 ml Dimethylsulfoxid wird unter Rühren und Einleiten von Argon mit 4,65 g pulverisiertem Kaliumhydroxid (90 %) und 9,45 ml Benzylbromid versetzt. Die Suspension wird 15 Stunden bei Raumtemperatur gerührt, nochmals mit 3,0 g Kaliumhydroxid und 6,3 ml Benzylbromid versetzt und weitere drei Stunden gerührt. Anschliessend giesst man die Mischung auf 1000 ml Eiswasser und extrahiert mit 500 ml Ethylacetat. Die organische Phase wird zweimal mit je 100 ml Wasser gewaschen, über Magnesiumsulfat getrocknet und unter 11 Torr bei 40° eingedampft. Den Rückstand kristallisiert man aus Ether-Petrolether. Der o-[N-Benzyl-(2,6-dichlorphenyl)-amino]-benzaldehyd schmilzt bei 136-138°.

b) Eine Suspension von 20,0 g Triphenylphosphin in 15 ml Toluol wird mit 12,82 g 3-Brompropionsäure versetzt. Man erhitzt das Gemisch unter Rühren während 15 Stunden unter Rückfluss, kühlt ab und kristallisiert das erstarrte Gemisch aus Ethylacetat-Methylenchlorid. Das 2-Carboxyethyltriphenylphosphoriumbromid schmilzt bei 194-196°.

c) Zu einer Lösung von 26,96 g 2-Carboxyethyltriphenylphosphoriumbromid in 78 ml Dimethylsulfoxid und 78 ml wasserfreiem Tetrahydrofuran setzt man unter Rühren bei 12° 0,25 g Natriumhydrid-Mineralöldispersion (55 %) zu. Die Suspension wird während 10 Minuten bei 15° gerührt, auf 0° abgekühlt und mit 23,15 g o-[N-Benzyl-(2,6-dichlorphenyl)-amino]-benzaldehyd versetzt. Die Suspension wird unter Rühren im Verlauf von 1 Stunde auf 40° erwärmt, 20 Minuten bei 40° gerührt und anschliessend auf 2,5 Liter Eiswasser gegossen. Die Suspension wird über Super Cel filtriert und das Filtrat bei 0° mit konzentrierter Salzsäure angesäuert. Die Suspension wird mit 1000 ml Ether extrahiert. Die organische Phase wird abgetrennt und dreimal mit je 100 ml 1-n Natronlauge und Wasser extrahiert. Die vereinigten wässrigen Extrakte werden mit konzentrierter Salzsäure angesäuert. Die ausgeschiedenen Kristalle werden mit 200 ml Ether extrahiert. Nach Waschen mit 30 ml Wasser und Trocknen über Magnesiumsulfat wird die Etherphase unter 11 Torr eingeengt, wobei die 4-[o-[N-Benzyl-(2,6-dichlorphenyl)-amino]-phenyl]-3-butensäure auskristallisiert, Smp. 166-168°.

d) 10,5 g 4-[o-[N-Benzyl-(2,6-dichlorphenyl)-amino]-phenyl]-3-butensäure werden in 200 ml Toluol gelöst und nach Zusatz von 6,5 g o-Dichlorbenzol und 3,0 g Palladium-Kohle (5 %) bei 40-45° hydriert. Nach 9 Stunden Hydrierdauer erhitzt man die Lösung auf 90° und filtriert vom Katalysator heiss ab. Dieser wird mit 300 ml heissem Toluol nachgewaschen. Das Filtrat wird unter 11 Torr bei 50° eingeengt, wobei die 4-[o-[(2,6-Dichlorphenyl)-amino]-phenyl]buttersäure auskristallisiert, Smp. 138-140°.

Beispiel 7:

Eine Lösung von 0,4 g [o-[(2,6-Dichlorphenyl)-N-benzyl-amino]-phenyl]-aceto-N-methyl-hydroxamsäure in 8,0 ml Tetrahydrofuran wird nach Zusatz von 0,283 g o-Dichlorbenzol und 0,08 g Palladiumkohle (5 %) bei Raumtemperatur hydriert. Nach 1 Stunde werden weitere 0,08 g Katalysator zugesetzt und die Hydrierung wird fortgesetzt. Nach insgesamt 3 Stunden wird vom Katalysator abfiltriert und das Filtrat unter 11 Torr bei 40° zur Trockne eingedampft. Der Rückstand kristallisiert aus Ether-Petrolether. Die [o-[(2,6-Dichlorphenyl)-amino]-phenyl]-aceto-N-methyl-hydroxamsäure schmilzt bei 135-137°.

Beispiel 8:

Zu einer Natriummethylat-Lösung, hergestellt aus 0,75 g Natrium und 15 ml wasserfreiem Methanol, wird unter Rühren eine Lösung von 0,84 g N-Methyl-hydroxylamin-hydrochlorid in 10 ml wasserfreiem Methanol zugesetzt. Die Suspension wird unter Rühren mit einer Lösung von 3,1 g o-[(2,6-Dichlorphenyl)-amino]-phenylessigsäuremethylester (beschrieben in Franz. Pat. 1 517 251) versetzt und anschliessend 20 Minuten auf 80° erhitzt. Man kühlt ab und engt unter 11 Torr bei 40° zur Trockne ein. Den Rückstand schüttelt man mit 50 ml Ether und 10 ml 2-n Salzsäure. Die organische Phase wird abgetrennt, mit 10 ml Wasser gewaschen, über Magnesiumsulfat getrocknet und unter 11 Torr bei 40° eingedampft. Der Rückstand, ein braunes Oel, wird flashchromatographiert an 500 g Kieselgel. Eluationsmittel Chloroform-Methanol 9:1. Die Fraktionen 5-8 werden vereinigt und unter 11 Torr bei 40° eingedampft. Den Rückstand kristallisiert man aus Ether-Petrolether. Die [o-[(2,6-Dichlorphenyl)-amino]-phenyl]-aceto-N-methyl-hydroxamsäure schmilzt bei 135-137°.

Beispiel 9:

In analoger Weise wie in den Beispielen 1 bis 8 beschrieben kann man herstellen:
[o-[(2,6-Dichlor-phenyl)-amino]-phenyl]-aceto-N-ethyl-hydroxamsäure;
[o-[(2,6-Dichlor-phenyl)-amino]-phenyl]-aceto-N-isopropyl-hydroxamsäure;
[o-[(3,5-Dichlor-phenyl)-amino]-phenyl]-aceto-N-methyl-hydroxamsäure; Smp.: 115 bis 117°;
[o-[(2,6-Dimethyl-phenyl)-amino]-phenyl]-aceto-N-methyl-hydroxamsäure;
[o-[(2-Chlor-6-methyl-phenyl)-amino]-phenyl]-aceto-N-methyl-hydroxamsäure; Smp.: 110 bis 111°;
[4-[(2, 6-Dichlor-phenyl)-amino]-thien-3-yl]-aceto-N-methyl-hydroxamsäure, Smp. 129-130°;
[o-[(2,6-Dichlor-phenyl)-N-methyl-amino]-phenyl]-aceto-N-methyl-hydroxamsäure;
[o-[(2,6-Dichlor-3-hydroxy-4-methoxy-phenyl)amino]phenyl]aceto-N-methyl-hydroxamsäure, Smp. 152 bis 157°; und
[o-(2,6-Dichlorphenyl)-N-benzyl-amino]cinnamo-N-methyl-hydroxamsäure, Smp. 197 bis 200°.

Beispiel 10:

In analoger Weise wie in Beispiel 1 beschrieben kann man herstellen: [m-[(2,6-Dichlorphenyl)-amino]-phenyl]-aceto-N-methyl-hydroxamsäure, Smp. 98-100° (aus Ether-Petrolether), ausgehend von m-[(2,6-Dichlorphenyl)-amino]-phenylessigsäure, Smp. 138-140° (aus Ether-Petrolether), und [p-[(2,6-Dichlorphenyl)-amino]-phenyl]-aceto-N-methyl-hydroxamsäure, Smp. 104-106° (aus Ether-Petrolether), ausgehend von p-[(2,6-Dichlorphenyl)-amino]-phenylessigsäure, Smp. 111-113° (aus Ether-Petrolether).

Die Ausgangsmaterialien können z.B. wie folgt hergestellt werden:

a) 25,0 g 3-[(2,6-Dichlorphenyl)-amino]-benzoesäure (beschrieben von A. Vedres et al., Acta Pharm. Hung. 1973, 43 (3-4), 152; C.A. 80/05-26876s) werden in 70 ml Methanol gelöst und unter Rühren mit 15 ml konzentrierter Schwefelsäure versetzt. Die Mischung wird 12 Stunden unter Rückfluss erhitzt, abgekühlt und unter 11 Torr bei 40° auf ein Volumen von ca. 20 ml eingedampft. Die Mischung wird auf 300 ml Eiswasser gegossen und das ausgeschiedene Oel mit 200 ml Ethylacetat extrahiert. Die organische Phase wird mit 50 ml Wasser, 50 ml 2-n. Natriumcarbonat und 50 ml Sole gewaschen, über Magnesiumsulfat getrocknet und unter 11 Torr bei 50° zur Trockne eingedampft. Der 3-[(2,6-Dichlorphenyl)-amino]-benzoesäuremethylester liegt als bräunliches Oel vor.

Analog kann man herstellen:
4-[(2,6-Dichlorphenyl)-amino]-benzoesäuremethylester, Smp. 158-160° (aus Ether-Petrolether), ausgehend von 4-[(2,6-Dichlorphenyl)-aminobenzoesäure, Smp. 184-186° (aus Ethanol) (siehe C.A. 80/05-26876s).

b) Eine Lösung von 13,8 g 3-[(2,6-Dichlorphenyl)-amino]-benzoesäure-methylester in 80 ml wasserfreiem Tetrahydrofuran wird unter Rühren und Einleiten von Stickstoff bei 40-50° zu einer Suspension von 5,0 g Lithiumaluminiumhydrid in 50 ml wasserfreiem Tetrahydrofuran zugetropft. die Mischung wird 45 Minuten unter Rückfluss gerührt, abgekühlt und bei 0-5° tropfenweise mit 50 ml Wasser, 50 ml 2N-Natronlauge und nochmals mit 100 ml Wasser versetzt. Die wässrige Mischung wird filtriert und das Nutschgut mit 50 ml Tetrahydrofuran nachgewaschen. Das Filtrat wird unter 11 Torr bei 40° eingedampft. Den Rückstand löst man in 100 ml Ethylacetat. Die organische Phase wird zweimal mit 30 ml Wasser, 20 ml 1N-Salzsäure und mit 30 ml Sole gewaschen, über Magnesiumsulfat getrocknet und unter 11 Torr bei 50° zur Trockne eingedampft. Den Rückstand kristallisiert man aus Ethylacetat. Der 3-[(2,6-Dichlorphenyl)-amino]-benzylalkohol schmilzt bei 148-150°.

Analog kann man herstellen:
4-[(2,6-Dichlorphenyl)-amino]-benzylalkohol, Smp. 93-95° (aus Ether-Petrolether), ausgehend von 4-[(2,6-Dichlorphenyl)-amino]-benzoesäure-methylester.

c) 9,0 g 3-[(2,6-Dichlorphenyl)-amino]-benzylalkohol werden unter Rühren bei Raumtemperatur in einer Argonatmosphäre mit 45,0 ml Phosphortribromid versetzt. Die Suspension wird bei Raumtemperatur 15 Stunden unter Argon gerührt und auf 700 ml Eis-Wasser gegossen. Die Mischung wird durch Zugabe von Natriumhydrogencarbonat neutralisiert (pH 7,0) und mit 700 ml Ethylacetat extrahiert. Die organische Phase wird abgetrennt, zweimal mit je 100 ml Wasser gewaschen, über Magnesiumsulfat getrocknet und unter 11 Torr bei 50° zur Trockne eingedampft. Den Rückstand kristallisiert man aus Ether-Petrolether. Das 3-[(2,6-Dichlorphenyl)-amino]-benzylbromid schmilzt bei 61-62°.

d) 14,0 g 4-[(2,6-Dichlorphenyl)-amino]-benzylalkohol werden bei Raumtemperatur mit 70 ml einer mit Salzsäuregas gesättigten Etherlösung versetzt. Die Lösung wird im verschlossenen Kolben 20 Minuten bei Raumtemperatur geschüttelt und unter 11 Torr bei 40° zur Trockne eingedampft. Der Rückstand, das 4-[-(2,6-Dichlorphenyl)-amino]-benzylchlorid liegt als Oel vor.

e) Eine Lösung von 9,9 g Natriumcyanid in 100 ml Dimethylsulfoxid wird unter Rühren bei 40-45° portionenweise mit 9,9 g 3-[(2,6-Dichlorphenyl)-amino]-benzylbromid versetzt. Die Mischung wird 30 Minuten bei Raumtemperatur gerührt und auf 800 ml Eis-Wasser gegossen. Die wässrige Suspension wird zweimal mit je 500 ml Ethylacetat extrahiert. Die organischen Extrakte werden vereinigt und mit 200 ml Wasser, 100 ml 1N-Salzsäure und 200 ml Sole gewaschen, über Magnesiumsulfat getrocknet und unter 11 Torr bei 50° zur Trockne eingedampft. Der Rückstand, das 3-[(2,6-Dichlorphenyl)-amino]-phenylacetonitril, wird aus Ether-Petrolether kristallisiert. Smp. 72-74°.

Analog kann man herstellen:

4-[(2,6-Dichlorphenyl)-amino]-phenylacetonitril, Smp. 164-166° (aus Ether-Petrolether), ausgehend von 4-[-(2,6-Dichlorphenyl)-amino]-benzylchlorid.

f) In eine Lösung von 7,7 g 3-[(2,6-Dichlorphenyl)-amino]-phenylacetonitril in 90 ml wasserfreiem Ether und 60 ml wasserfreiem Ethanol wird bei 0° Salzsäuregas bis zur Sättigung eingeleitet. Unter Kühlung wird sodann weitere 4 Stunden Salzsäuregas eingeleitet. Die Mischung wird 15 Stunden bei Raumtemperatur gerührt und unter 11 Torr bei 50° zur Trockne eingedampft. Den Rückstand versetzt man mit 100 ml Wasser und 200 ml Ether und erhitzt das Gemisch bei einer Badtemperatur von 60° unter Rückfluss während 5 Stunden. Man kühlt ab, trennt die organische Phase ab, wäscht sie zweimal mit je 50 ml Wasser, trocknet sie über Magnesiumsulfat und engt sie unter 11 Torr bei 40° zur Trockne ein. Den Rückstand löst man in 100 ml 5%iger ethanolischer Kaliumhydroxid-Lösung. Die Lösung wird bei Raumtemperatur unter Rühren tropfenweise mit 20 ml Wasser versetzt und 8 Stunden bei Raumtemperatur gerührt, wobei das Kaliumsalz der m-[(2,6-Dichlorphenyl)-amino]-phenylessigsäure auskristallisiert. Die Kristalle werden abfiltriert und unter Rühren mit 15 ml 2N-Salzsäure versetzt. Die Suspension wird mit 100 ml Ether extrahiert. Man wäscht die organische Phase mit 20 ml Wasser, trocknet sie über Magnesiumsulfat und engt sie unter 11 Torr bei 40° zur Trockne ein. Den Rückstand kristallisiert man aus Ether-Petrolether. Die m-[(2,6-Dichlorphenyl)-amino]-phenylessigsäure schmilzt bei 138-140°.

Analog kann man herstellen:

p-[(2,6-Dichlorphenyl)-amino]-phenylessigsäure, Smp. 111-113° (aus Ether-Petrolether).

Beispiel 11:

Eine Suspension von 2,1 g o-[(2,6-Dichlorphenyl)-amino]-phenylessigsäure-p-nitrophenylester (beschrieben in DE-OS 2144641) und 0,42 g N-Methyl-hydroxylamin-hydrochlorid in 25 ml absolutem Chloroform wird bei Raumtemperatur mit 1,38 ml Triethylamin versetzt. Die klare Lösung wird 3 Stunden bei Raumtemperatur gerührt, mit 25 ml Chloroform verdünnt und mit 20 ml 1N-Salzsäure, 20 ml Wasser und dreimal mit 20 ml Sole gewaschen, über Magnesiumsulfat getrocknet und unter 11 Torr zur Trockne eingedampft. Den Rückstand chromatographiert man an 70 g Silicagel. Die Fraktionen 1-8, eluiert mit je 200 ml Methylenchlorid, werden verworfen. Die Fraktionen 9-15, eluiert mit je 200 ml Ethylacetat, werden vereinigt und unter 11 Torr bei 50° eingedampft. Den Rückstand kristallisiert man aus Ether-Petrolether. Die [o-[(2,6-Dichlorphenyl)-amino]-phenyl]-aceto-N-methyl-hydroxamsäure schmilzt bei 135-137°.

Beispiel 12:

Eine Mischung von 5,2 g o-[(2,6-Dichlorphenyl)-amino]-phenylessigsäure-cyanmethylester (beschrieben in DE-OS 2144641) und 1,32 g N-Methylhydroxylamin-hydrochlorid in 40 ml Acetonitril wird bei Raumtemperatur mit 1,58 ml Triethylamin versetzt. Die Mischung wird 8 Stunden bei Raumtemperatur gerührt, mit 2 Tropfen Eisessig versetzt und weitere 3 Stunden bei Raumtemperatur gerüht. Hierauf wird die Mischung unter 11 Torr bei 50° eingedampft. Den Rückstand versetzt man mit 30 ml Wasser, 5 ml 1N-Salzsäure und 100 ml Ethylacetat. Die Ethylacetatlösung wird abgetrennt und die wässrige Phase nochmals mit 100 ml Ethylacetat geschüttelt. Die vereinten organischen Phasen werden mit 30 ml 1N-Salzsäure, 40 ml Wasser, 30 ml 2N-Natriumcarbonatlösung und mit 40 ml Wasser gewaschen, über Magnesiumsulfat getrocknet und unter 11 Torr bei 50° eingedampft. Den Rückstand chromatographiert man an 60 g Silicagel. Die Fraktionen 1-4, eluiert mit je 200 ml Hexan-Ethylacetat (1:1), werden verworfen. Die Fraktionen 5-7, eluiert mit je 200 ml des gleichen Gemisches, werden vereinigt und unter 11 Torr bei 50° eingedampft. Den Rückstand kristallisiert man aus Ethylacetat-Petrolether. Die [o-[(2,6-Dichlorphenyl)-amino]-phenyl]-aceto-N-methyl-hydroxamsäure schmilzt bei 135-137°.

Beispiel 13:

Eine Lösung von 1,97 g o-[(2,6-Dichlorphenyl)-amino]-phenylessigsäure-N-succinimidester in 15 ml Dimethylsulfoxid wird bei Raumtemperatur unter Rühren mit 0,6 g N-Methylhydroxylamin-hydrochlorid und 1,38 ml Triethylamin versetzt. Die Mischung wird 8 Stunden bei Raumtemperatur gerührt und auf 300 ml Wasser gegossen. Die Suspension wird mit 200 ml Ethylacetat extrahiert. Die organische Phase wird abgetrennt und mit 40 ml 1N-Salzsäure, 40 ml Wasser, 40 ml 2N-Natriumcarbonatlösung und mit 40 ml Wasser gewaschen, über Magnesiumsulfat getrocknet und unter 11 Torr bei 50° eingedampft. Den Rückstand kristallisiert man aus Ether-Petrolether. Die [o-[(2,6-Dichlorphenyl)-amino]-phenyl]-aceto-N-methyl-hydroxamsäure schmilzt bei 135-137°.

Das Ausgangsmaterial z.B. kann auf folgende Weise hergestellt werden: Eine Lösung von 17,8 g o-[-(2,6-Dichlorphenyl)-amino]-phenylessigsäure in 100 ml absolutem Tetrahydrofuran wird bei Raumtemperatur unter Rühren mit 7,35 g N-Hydroxysuccinimid und 14,3 g N,N′-Dicyclohexylcarbodiimid versetzt. Die Mischung wird 20 Minuten bei Raumtemperatur gerührt und filtriert. Das Filtrat wird unter 11 Torr bei 50° eingeengt und mit 50 ml Ether versetzt. Die ausgeschiedenen Kristalle werden abfiltriert und mit Ether gewaschen. Der o-[(2,6-Dichlorphenyl)-amino]-phenylessigsäure-N-succinimidester schmilzt bei 136°.

Beispiel 14:

2,0 g [o-[(2,6-Dichlorphenyl)amino]phenyl]aceto-N-methyl-hydroxamsäure werden unter Erwärmen auf 40° in 10 ml Eisessig gelöst. Die Lösung wird auf Raumtemperatur abgekühlt, unter Rühren mit 1,0 ml Acetanhydrid versetzt, anschliessend 15 Stunden bei Raumtemperatur gerührt und bei 11 Torr und 50° eingedampft. Den Rückstand versetzt man mit 50 ml Eiswasser und extrahiert die Suspension mit 100 ml Ethylacetat. Die organische Phase wird mit 20 ml Wasser und 20 ml gesättigter Natriumchloridlösung gewaschen, über Magnesiumsulfat getrocknet und bei 11 Torr und 50° eingedampft. Den Rückstand Kristallisiert man aus Ether-Petrolether. Die [o-(2,6-Dichlorphenyl)amino]phenyl]-aceto-N-methyl-o-acetyl-hydroxamsäure schmilzt bei 99-100°.

Beispiel 15:

3,25 g [o-[(2,6-Dichlorphenyl)amino]-phenyl]aceto-N-methyl-hydroxamsäure werden unter Erwärmen auf 40° in einer Mischung aus 230,0 ml absolutem Benzol und 3,74 ml Pyridin gelöst. Die Lösung wird unter Rühren bei 20° mit 1,69 ml Pivaloylchlorid und 2,58 ml Triethylamin versetzt und das Gemisch 10 Minuten bei 20° gerührt bei 11 Torr und 50° eingedampft. Der Rückstand wird mit 50 ml Wasser, 5,0 ml 1N-Salzsäure und 100 ml Ether geschüttelt. Die organische Phase wird abtgetrennt, mit 10 ml 0,1N-Salzsäure und 20 ml Wasser gewaschen, über Magnesiumsulfat getrocknet bei 11 Torr und 40° eingedampft. Den Rückstand Kristallisiert man aus Ether-Petrolether. Die [o-[(2,6-Dichlorphenyl)-amino]-phenyl]-aceto-N-methyl-o-pivaloyl-hydroxamsäure schmilzt bei 107-110°.

Beispiel 16:

Tabletten, enthaltend je 50 mg Wirkstoff, z.B. [o-[(2,6-Dichlorphenyl)-amino]-phenyl]-aceto-N-methyl-hydroxamsäure, können wie folgt hergestellt werden:

| Zusammensetzung (für 10000 Tabletten) | |
|---|---|
| Wirkstoff | 500.0 g |
| Lactose | 500.0 g |
| Kartoffelstärke | 352.0 g |
| Gelatine | 8.0 g |
| Talk | 60.0 g |
| Magnesiumstearat | 10.0 g |
| Siliciumdioxid (hochdispers) | 20.0 g |
| Ethanol | q.s. |

Der Wirkstoff wird mit der Lactose und 292 g Kartoffelstärke vermischt, die Mischung mit einer alkoholischen Lösung der Gelatine befeuchtet und durch ein Sieb granuliert. Nach dem Trocknen mischt man den Rest der Kartoffelstärke, den Talk, das Magnesiumstearat und das hochdisperse Siliciumdioxid zu und presst das Gemisch zu Tabletten von je 145,0 mg Gewicht und 50,0 mg Wirkstoffgehalt, die gewünschtenfalls mit Teilkerben zur feineren Anpassung der Dosierung versehen sein können.

Beispiel 17:

Lacktabletten, enthaltend je 100 mg Wirkstoff, z.B. [o-[(2,6-Dichlorphenyl)-amino]-phenyl]-aceto-N-methyl-hydroxamsäure, können wie folgt hergestellt werden:

| Zusammensetzung (für 1000 Tabletten) | |
|---|---|
| Wirkstoff | 100.00 g |
| Lactose | 100.00 g |
| Maisstärke | 70.00 g |
| Talk | 8.50 g |
| Calciumstearat | 1.50 g |
| Hydroxypropylmethylcellulose | 2.36 g |
| Schellack | 0.64 g |
| Wasser | q.s. |
| Dichlormethan | q.s. |

Der Wirkstoff, die Lactose und 40 g der Maisstärke werden gemischt und mit einem Kleister, hergestellt aus 15 g Maisstärke und Wasser (unter Erwärmen), befeuchtet und granuliert. Das Granulat wird getrocknet, der Rest der Maisstärke, der Talk und das Calciumstearat werden zugegeben und mit dem Granulat vermischt. Das Gemisch wird zu Tabletten (Gewicht: 280 mg) verpresst und diese mit einer Lösung der Hydroxypropylmethylcellulose und des Schellacks in Methylenchlorid lackiert; Endgewicht der Lacktablette: 283 mg.

Beispiel 18:

In analoger Weise wie in den Beispielen 16 und 17 beschrieben können auch Tabletten und Lacktabletten, enthaltend eine andere Verbindung I oder ein pharmazeutisch verwendbares Salz einer Verbindung I, z.B. gemäss den Beispielen 1 bis 15, hergestellt werden.

**Ansprüche**

1. Eine Verbindung der Formel

$$Ar_1 - \underset{R_1}{N} - Ar_2 - Z - CO - \underset{R_2}{N} - O - R_3 \qquad (I),$$

worin $Ar_1$ einen unsubstituierten oder substituierten Arylrest bedeutet, $Ar_2$ einen unsubstituierten oder substituierten Arylenrest bedeutet, $R_1$ Wasserstoff oder einen aliphatischen oder araliphatischen Rest bedeutet, $R_2$ einen aliphatischen oder araliphatischen Rest bedeutet, $R_3$ Wasserstoff, Niederalkyl oder Niederalkanoyl darstellt und Z einen zweiwertigen aliphatischen Rest bedeutet.

2. Eine Verbindung gemäss Anspruch 1 der Formel I, worin $Ar_1$ einen unsubstituierten oder substituierten Arylrest bedeutet, $Ar_2$ einen unsubstituierten oder substituierten Arylenrest bedeutet, $R_1$ Wasserstoff oder einen aliphatischen oder araliphatischen Rest bedeutet, $R_2$ einen aliphatischen oder araliphatischen Rest bedeutet, $R_3$ Wasserstoff ist und Z einen zweiwertigen aliphatischen Rest bedeutet.

3. Eine Verbindung gemäss Anspruch 1 der Formel I, worin $Ar_1$ unsubstituiertes oder durch Substituenten ausgewählt aus der Gruppe bestehend aus Niederalkyl, Niederalkoxy, Halogen, Hydroxy, Trifluormethyl und Phenylniederalkoxy, wobei in Phenylniederalkoxy Phenyl unsubstituiert oder ein-oder mehrfach durch Substituenten augewählt aus der Gruppe bestehend aus Niederalkyl, Niederalkoxy, Halogen, Hydroxy und Trifluormethyl substituiert ist, ein- oder mehrfach substituiertes Phenyl bedeutet, $Ar_2$ einen unsubstituierten oder ein- oder mehrfach durch Substituenten ausgewählt aus der Gruppe bestehend aus Niederalkyl, Niederalkoxy, Trifluormethyl, Halogen und Hydroxy substituierten Phenylen- oder Thienylenrest bedeutet, $R_1$ Wasserstoff, Niederalkyl, Niederalkenyl oder Phenylniederalkyl, welches im Phenylteil unsubstituiert oder durch Substituenten ausgewählt aus der Gruppe bestehend aus Niederalkyl, Niederalkoxy, Halogen, Hydroxy und Trifluormethyl ein- oder mehrfach substituiert ist, darstellt, $R_2$ Niederalkyl, Niederalkenyl oder Phenylniederalkyl, welches im Phenylteil unsubstituiert oder durch Substituenten ausgewählt aus der Gruppe bestehend aus Niederalkyl, Niederalkoxy, Halogen, Hydroxy und Trifluormethyl ein- oder mehrfach substituiert ist, bedeutet, $R_3$ Wasserstoff, Niederalkyl oder Niederalkanoyl darstellt und Z Niederalkylen, Niederalkyliden oder Niederalkenylen ist.

4. Eine Verbindung gemäss Anspruch 1 der Formel I, worin $Ar_1$ unsubstituiertes oder durch Substituenten ausgewählt aus der Gruppe bestehend aus Niederalkyl, Niederalkoxy, Halogen, Hydroxy, Trifluormethyl und Phenylniederalkoxy, wobei Phenyl unsubstituiert oder ein- oder mehrfach durch Substituenten ausgewählt aus der Gruppe bestehend aus Niederalkyl, Niederalkoxy, Halogen, Hydroxy und Trifluormethyl substituiert sein kann, substituiertes Phenyl bedeutet, $Ar_2$ einen unsubstituierten oder durch Substituenten ausgewählt aus der Gruppe bestehend aus Niederalkyl, Niederalkoxy, Trifluormethyl, Halogen und Hydroxy substituierten Phenylen- oder Thienylenrest bedeutet, $R_1$ Wasserstoff, Niederalkyl, Niederalkenyl oder Phenylniederalkyl, welches im Phenylteil unsubstituiert oder durch Substituenten ausgewählt aus der Gruppe bestehend aus Niederalkyl, Niederalkoxy, Halogen, Hydroxy und Trifluormethyl substituiert ist, darstellt, $R_2$ Niederalkyl, Niederalkenyl oder Phenylniederalkyl, welches im Phenylteil unsubstituiert oder durch Substituenten ausgewählt aus der Gruppe bestehend aus Niederalkyl, Niederalkoxy, Halogen, Hydroxy und Trifluormethyl substituiert ist, darstellt, $R_3$ Wasserstoff ist und Z Niederalkylen, Niederalkyliden oder Niederalkenylen bedeutet.

5. Eine Verbindung gemäss Anspruch 1 der Formel I, worin $Ar_1$ unsubstituiertes oder durch Substituenten ausgewählt aus der Gruppe bestehend aus Niederalkyl mit bis und mit 7 C-Atomen, Niederalkoxy mit bis und mit 7 C-Atomen, Halogen mit einer Atomnummer bis und mit 35, Hydroxy, Trifluormethyl und 1-Phenylniederalkoxy mit bis und mit 4 C-Atomen im Niederalkoxyteil substituiertes Phenyl bedeutet, $Ar_2$ einen unsubstituierten oder durch Substituenten ausgewählt aus der Gruppe bestehend aus Niederalkyl mit bis und mit 7 C-Atomen, Halogen mit einer Atomnummer bis und mit 35 und Hydroxy substituierten Phenylen- oder Thienylenrest bedeutet, $R_1$ Wasserstoff, Niederalkyl mit bis und mit 7 C-Atomen, Niederalkenyl mit 3 der 4 C-Atomen oder Phenylniederalkyl, welches bis und mit 7 C-Atome im Niederalkylteil aufweist und dessen Phenylteil unsubstituiert oder durch Substituenten ausgewählt aus der Gruppe bestehend aus Niederalkyl mit bis und mit 7 C-Atomen, Niederalkoxy mit bis und mit 7 C-Atomen, Halogen mit einer Atomnummer bis und mit 35, Hydroxy und Trifluormethyl substituiert ist, darstellt $R_2$ Niederalkyl mit bis und mit 7 C-Atomen, Niederalkenyl mit 3 oder 4 C-Atomen oder Phenylniederalkyl, welches bis und mit 7 C-Atome im Niederalkylteil aufweist und dessen Phenylteil unsubstituiert oder durch Substituenten ausgewählt aus der Gruppe bestehend aus Niederalkyl mit bis und mit 7 C-Atomen, Niederalkoxy mit bis und mit 7 C-Atomen, Halogen mit einer Atomnummer bis und mit 35, Hydroxy und Trifluormethyl substituiert ist, darstellt, $R_3$ Wasserstoff ist und Z Niederalkylen mit bis und mit 4 C-Atomen, Niederalkyliden mit 2 bis und mit 4 C-Atomen oder Niederalkenylen mit 2 bis und mit 4 C-Atomen bedeutet.

6. Eine Verbindung gemäss Anspruch 1 der Formel I, worin $Ar_1$ durch Niederalkyl mit bis und mit 4 C-Atomen, Niederalkoxy mit bis und mit 4 C-Atomen, Halogen mit einer Atomnummer bis und mit 35, Hydroxy und/oder Benzyloxy substituiertes Phenyl bedeutet, $Ar_2$ einen unsubstituierten oder durch Hydroxy

substituierten Phenylen- oder Thienylenrest bedeutet, $R_1$ Wasserstoff oder 1-Phenylniederalkyl mit bis und mit 4 C-Atomen im Niederalkylteil bedeutet, $R_3$ Wasserstoff ist, $R_2$ Niederalkyl mit bis und mit 4 C-Atomen bedeutet und Z Niederalkylen mit bis und mit 4 C-Atomen oder Niederalkenylen mit 2 bis und mit 4 C-Atomen bedeutet.

7. Eine Verbindung gemäss Anspruch 1 der Formel I, worin $Ar_1$ durch Niederalkyl mit bis und mit 4 C-Atomen, Niederalkoxy mit bis und mit 4 C-Atomen, Halogen mit einer Atomnummer bis und mit 35, Hydroxy und/oder Benzyloxy ein-, zwei-, drei- oder vierfach substituiertes Phenyl bedeutet, $Ar_2$ unsubstituiertes 1,2-, 1,3- oder 1,4-Phenylen oder unsubstituiertes 3,4-Thienylen bedeutet, $R_1$ Wasserstoff, 1-Phenylniederalkyl mit bis und mit 4 C-Atomen im Niederalkylteil oder $C_1$-$C_4$-Alkyl ist, $R_2$ $C_1$-$C_4$-Alkyl darstellt, $R_3$ Wasserstoff oder $C_2$-$C_5$-Alkanoyl ist und Z $C_1$-$C_4$-Alkylen oder $C_2$-$C_4$-Alkenylen bedeutet.

8. Eine Verbindung gemäss Anspruch 1 der Formel I, worin $Ar_1$ durch Halogen mit einer Atomnummer bis und mit 35 in den Positionen 2 und 6 zweifach substituiertes Phenyl bedeutet, $Ar_2$ 1,2-Phenylen bedeutet, $R_1$ Wasserstoff oder Benzyl bedeutet, $R_2$ Niederalkyl mit bis und mit 4 C-Atomen bedeutet, $R_3$ Wasserstoff ist und Z Methylen oder Ethylen bedeutet.

9. Eine Verbindung gemäss Anspruch 1 der Formel I, worin $Ar_1$ 2,6-Difluorphenyl oder 2,6-Dichlorphenyl ist, $Ar_2$ unsubstituiertes 1,2- oder 1,4-Phenylen oder unsubstituiertes 3,4-Thienylen darstellt, $R_1$ Wasserstoff oder Benzyl bedeutet, $R_2$ $C_1$-$C_4$-Alkyl ist, $R_3$ Wasserstoff ist und Z $C_1$-$C_4$-Alkylen bedeutet.

10. Eine Verbindung gemäss Anspruch 1 der Formel I, worin $Ar_1$ 2,6-Dichlorphenyl ist, $Ar_2$ unsubstituiertes 1,2-Phenylen oder unsubstituiertes 3,4-Thienylen ist, $R_1$ Wasserstoff ist, $R_2$ $C_1$-$C_4$-Alkyl ist, $R_3$ Wasserstoff ist und Z Methylen bedeutet.

11. [o-[(2,6-Dichlorphenyl)-amino]-phenyl]-aceto-N-methyl-hydroxamsäure.

12. 4-[o-[(2,6-Dichlorphenyl)-amino]-phenyl]-butyro-N-methyl-hydroxamsäure.

13. [4-[(2,6-Dichlor-phenyl)-amino]-thien-3-yl]-aceto-N-methyl-hydroxamsäure.

14. [m-[(2,6-Dichlorphenyl)-amino]phenyl]aceto-N-methyl-hydroxamsäure.

15. [p-[(2,6-Dichlorphenyl)-amino]-phenyl]-aceto-N-methyl-hydroxamsäure.

16. [o-[(2,6-Dichlor-4-benzyloxy-3-methoxy-phenyl)-amino]-phenyl]-aceto-N-methyl-hydroxamsäure, [o-[-(2,6-Dichlor-4-hydroxy-3-methoxy-phenyl)-amino]-phenyl]-aceto-N-methyl-hydroxamsäure, [o-[(2,6-Dichlorphenyl)-N-benzyl-amino]-phenyl]-aceto-N-methyl-hydroxamsäure, [o-(2,6-Dichlorphenyl)-amino]-cinnamo-N-methyl-hydroxamsäure, [o-[(2,6-Difluorphenyl)-amino]-phenyl]-aceto-N-methyl-hydroxamsäure, 3-[o-[(2,6-Dichlorphenyl)- amino]-phenyl]-propio-N-methyl-hydroxamsäure, [o-[(2,6-Dichlor-phenyl)-amino]-phenyl]-aceto-N-ethyl-hydroxamsäure, [o-[(2,6-Dichlor-phenyl)-amino]-phenyl]-aceto-N-isopropyl-hydroxamsäure, [o-[(3,5-Dichlor-phenyl)-amino]-phenyl]-aceto-N-methyl-hydroxamsäure, [o-[(2,6-Dimethyl-phenyl)-amino]-phenyl]-aceto-N-methyl-hydroxamsäure, [o-[(2-chlor-6-methyl-phenyl)-amino]-phenyl]-aceto-N-methyl-hydroxamsäure oder [o-[(2,6-Dichlor-phenyl)-N-methyl-amino]-phenyl]-aceto-N-methyl-hydroxamsäure.

17. [o-[(2,6-Dichlor-3-hydroxy-4-methoxy-phenyl)amino]phenyl]aceto-N-methyl-hydroxamsäure, [o-(2,6-Dichlorphenyl)-N-benzyl-amino]cinnamo-N-methyl-hydroxamsäure, [o-(2,6-Dichlorphenyl)amino]phenyl]-aceto-N-methyl-o-acetyl-hydroxamsäure oder [o-[(2,6-Dichlorphenyl)-amino]-phenyl]-aceto-N-methyl-o-pivaloyl-hydroxamsäure.

18. Eine Verbindung gemäss einem der Ansprüche 1 bis 17 der Formel I in Form eines Salzes.

19. Eine Verbindung gemäss einem der Ansprüche 1 bis 18 der Formel I in Form eines pharmazeutisch verwendbaren Salzes.

20. Eine Verbindung gemäss einem der Ansprüche 1 bis 17 oder ein pharmazeutisch verwendbares Salz davon gemäss Anspruch 19 zur Anwendung in einem Verfahren zur therapeutischen Behandlung des menschlichen oder tierischen Körpers.

21. Eine Verbindung gemäss einem der Ansprüche 2, 4 bis 6, 8 und 11 bis 16 zur Anwendung in einem Verfahren zur therapeutischen Behandlung des menschlichen oder tierischen Körpers.

22. Eine Verbindung gemäss einem der Ansprüche 1 bis 17, 20 und 21 oder ein pharmazeutisch verwendbares Salz davon gemäss Anspruch 19 oder 20 zur Anwendung als Antiinflammatorikum und/oder Antiallergikum.

23. Eine Verbindung gemäss einem der Ansprüche 2, 4 bis 6, 8, 11 bis 16 und 21 zur Anwendung als Antiinflammatorikum und/oder Antiallergikum.

24. Ein pharmazeutisches Präparat, als Wirkstoff enthaltend eine Verbindung gemäss einem der Ansprüche 1 bis 17 und 20 bis 23 oder ein pharmazeutisch verwendbares Salz davon gemäss einem der Ansprüche 19, 20 und 22 gegebenenfalls neben üblichen pharmazeutischen Hilfsstoffen.

25. Ein pharmazeutisches Präparat, als Wirkstoff enthaltend eine Verbindung gemäss einem der Ansprüche 2, 4 bis 6, 8, 11 bis 16, 21 und 23 gegebenenfalls neben üblichen pharmazeutischen Hilfsstoffen.

26. Ein antiinflammatorisch und/oder antiallergisch wirksames pharmazeutisches Präparat gemäss

Anspruch 24 oder 25, dadurch gekennzeichnet, dass man einen antiinflammatorisch und/oder antiallergisch wirksamen Wirkstoff wählt.

27. Ein antiinflammatorisch und/oder antiallergisch wirksames pharmazeutisches Präparat gemäss Anspruch 25, dadurch gekennzeichnet, dass man einen antiinflammatorisch und/oder antiallergisch wirksamen Wirkstoff wählt.

28. Verfahren zur Herstellung einer Verbindung der Formel I gemäss einem der Ansprüche 1 bis 17 oder eines Salzes davon gemäss Anspruch 18, dadurch gekennzeichnet, dass man

a) eine Verbindung der Formel

$$Ar_1 - \underset{R_1}{N} - Ar_2 - Z - COOH \qquad (IIa)$$

oder ein Salz und/oder ein reaktionsfähiges Derivat davon mit einer Verbindung der Formel

$R_3$- O - HN - $R_2$ (IIb)

oder einem Salz davon umsetzt oder

b) zur Herstellung einer Verbindung I, worin $R_3$ Wasserstoff ist, oder eines Salzes davon in einer Verbindung der Formel

$$Ar_1 - \underset{R_1}{N} - Ar_2 - Z - CO - \underset{R_2}{N} - OP_1 \qquad (III)$$

oder einem Salz davon die Schutzgruppe $P_1$ in Wasserstoff überführt oder

c) eine Verbindung der Formel

$$Ar_1 - \underset{R_1}{N} - H \qquad (IVa)$$

oder ein Salz davon mit einer Verbindung der Formel

$$X_1 - Ar_2 - Z - CO - \underset{R_2}{N} - O - R_3 \qquad (IVb),$$

worin $X_1$ reaktionsfähiges verestertes Hydroxy bedeutet, oder einem Salz davon umsetzt und jeweils gewünschtenfalls eine verfahrensgemäss oder auf andere Weise erhältliche Verbindung I in eine andere Verbindung I überführt, ein verfahrensgemäss erhältliches Isomerengemisch in die Komponenten auftrennt, ein verfahrensgemäss erhältliches Enantiomeren-beziehungsweise Diastereomerengemisch in die Enantiomeren beziehungsweise Diastereomeren aufspaltet und/oder eine verfahrensgemäss erhältliche freie Verbindung I in ein Salz oder ein verfahrensgemäss erhältliches Salz in die freie Verbindung I oder in ein anderes Salz umwandelt.

29. Verfahren zur Herstellung eines pharmazeutischen Präparates gemäss Anspruch 24, dadurch gekennzeichnet, dass man den Wirkstoff, gegebenenfalls unter Beimischung von üblichen pharmazeutischen Hilfsstoffen, zu einem pharmazeutischen Präparat verarbeitet.

30. Verfahren gemäss Anspruch 29 zur Herstellung eines antiinflammatorisch und/oder antiallergisch wirksamen pharmazeutischen Präparates gemäss Anspruch 26, dadurch gekennzeichnet, dass man einen antiinflammatorisch und/oder antiallergisch wirksamen Wirkstoff wählt.

31. Verwendung einer Verbindung gemäss einem der Ansprüche 1 bis 17 und 20 bis 23 oder eines pharmazeutisch verwendbaren Salzes davon gemäss einem der Ansprüche 19, 20 und 22 zur Herstellung eines pharmazeutischen Präparats.

32. Verwendung einer Verbindung gemäss Anspruch 31 zur Herstellung eines Antiinflammatorikums und/oder Antiallergikums.

33. Verwendung einer Verbindung gemäss einem der Ansprüche 1 bis 17 und 20 bis 23 oder eines pharmazeutisch verwendbaren Salzes davon gemäss einem der Ansprüche 19, 20 und 22 zur Herstellung eines pharmazeutischen Präparats auf nicht-chemischem Wege.

Patentansprüche für folgende Vertragsstaaten: ES, GR

1. Verfahren zur Herstellung einer Verbindung der Formel

$$Ar_1 - \underset{R_1}{N} - Ar_2 - Z - CO - \underset{R_2}{N} - O - R_3 \qquad (I),$$

worin $Ar_1$ einen unsubstituierten oder substituierten Arylrest bedeutet, $Ar_2$ einen unsubstituierten oder substituierten Arylenrest bedeutet, $R_1$ Wasserstoff oder einen aliphatischen oder araliphatischen Rest bedeutet, $R_2$ einen aliphatischen oder araliphatischen Rest bedeutet, $R_3$ Wasserstoff, Niederalkyl oder Niederalkanoyl darstellt und Z einen zweiwertigen aliphatischen Rest bedeutet, oder eines Salzes davon, dadurch gekennzeichnet, dass man

a) eine Verbindung der Formel

$$Ar_1 - \underset{R_1}{N} - Ar_2 - Z - COOH \qquad (IIa)$$

oder ein Salz und/oder ein reaktionsfähiges Derivat davon mit einer Verbindung der Formel

$R_3$- O - HN - $R_2$ (IIb)

oder einem Salz davon umsetzt oder

b) zur Herstellung einer Verbindung I, worin $R_3$ Wasserstoff ist, oder eines Salzes davon in einer Verbindung der Formel

$$Ar_1 - \underset{R_1}{N} - Ar_2 - Z - CO - \underset{R_2}{N} - OP_1 \qquad (III)$$

oder einem Salz davon die Schutzgruppe $P_1$ in Wasserstoff überführt oder

c) eine Verbindung der Formel

$$Ar_1 - \underset{R_1}{N} - H \qquad (IVa)$$

oder ein Salz davon mit einer Verbindung der Formel

$$X_1 - Ar_2 - Z - CO - \underset{R_2}{N} - O - R_3 \qquad (IVb),$$

worin $X_1$ reaktionsfähiges verestertes Hydroxy bedeutet, oder einem Salz davon umsetzt und jeweils gewünschtenfalls eine verfahrensgemäss oder auf andere Weise erhältliche Verbindung I in eine andere Verbindung I überführt, ein verfahrensgemäss erhältliches Isomerengemisch in die Komponenten auftrennt, ein verfahrensgemäss erhältliches Enantiomeren-beziehungsweise Diastereomerengemisch in die Enantiomeren beziehungsweise Diastereomeren aufspaltet und/oder eine verfahrensgemäss erhältliche freie Verbindung I in ein Salz oder ein verfahrensgemäss erhältliches Salz in die freie Verbindung I oder in ein anderes Salz umwandelt.

2. Verfahren gemäss Anspruch 1, dadurch gekennzeichnet, dass man eine Verbindung der Formel I, worin $Ar_1$ einen unsubstituierten oder substituierten Arylrest bedeutet, $Ar_2$ einen unsubstituierten oder substituierten Arylenrest bedeutet, $R_1$ Wasserstoff oder einen aliphatischen oder araliphatischen Rest bedeutet, $R_2$ einen aliphatischen oder araliphatischen Rest bedeutet, $R_3$ Wasserstoff ist und Z einen zweiwertigen aliphatischen Rest bedeutet, herstellt.

3. Verfahren gemäss Anspruch 1, dadurch gekennzeichnet, dass man eine Verbindung der Formel I, worin $Ar_1$ unsubstituiertes oder durch Substituenten ausgewählt aus der Gruppe bestehend aus Niederalkyl, Niederalkoxy, Halogen, Hydroxy, Trifluormethyl und Phenylniederalkoxy, wobei in Phenylniederalkoxy Phenyl unsubstituiert oder ein-oder mehrfach durch Substituenten augewählt aus der Gruppe bestehend aus Niederalkyl, Niederalkoxy, Halogen, Hydroxy und Trifluormethyl substituiert ist, ein-oder mehrfach substituiertes Phenyl bedeutet, $Ar_2$ einen unsubstituierten oder ein- oder mehrfach durch Substituenten ausgewählt aus der Gruppe bestehend aus Niederalkyl, Niederalkoxy, Trifluormethyl, Halogen und Hydroxy substituierten Phenylen- oder Thienylenrest bedeutet, $R_1$ Wasserstoff, Niederalkyl, Niederalkenyl oder Phenylniederalkyl, welches im Phenylteil unsubstituiert oder durch Substituenten ausgewählt aus der

Gruppe bestehend aus Niederalkyl, Niederalkoxy, Halogen, Hydroxy und Trifluormethyl ein- oder mehrfach substituiert ist, darstellt, $R_2$ Niederalkyl, Niederalkenyl oder Phenylniederalkyl, welches im Phenylteil unsubstituiert oder durch Substituenten ausgewählt aus der Gruppe bestehend aus Niederalkyl, Niederalkoxy, Halogen, Hydroxy und Trifluormethyl ein- oder mehrfach substituiert ist, bedeutet, $R_3$ Wasserstoff, Niederalkyl oder Niederalkanoyl darstellt und Z Niederalkylen, Niederalkyliden oder Niederalkenylen ist, herstellt.

4. Verfahren gemäss Anspruch 1, dadurch gekennzeichnet, dass man eine Verbindung der Formel I, worin $Ar_1$ unsubstituiertes oder durch Substituenten ausgewählt aus der Gruppe bestehend aus Niederalkyl, Niederalkoxy, Halogen, Hydroxy, Trifluormethyl und Phenylniederalkoxy, wobei Phenyl unsubstituiert oder ein- oder mehrfach durch Substituenten ausgewählt aus der Gruppe bestehend aus Niederalkyl, Niederalkoxy, Halogen, Hydroxy und Trifluormethyl substituiert sein kann, substituiertes Phenyl bedeutet, $Ar_2$ einen unsubstituierten oder durch Substituenten ausgewählt aus der Gruppe bestehend aus Niederalkyl, Niederalkoxy, Trifluormethyl, Halogen und Hydroxy substituierten Phenylen- oder Thienylenrest bedeutet, $R_1$ Wasserstoff, Niederalkyl, Niederalkenyl oder Phenylniederalkyl, welches im Phenylteil unsubstituiert oder durch Substituenten ausgewählt aus der Gruppe bestehend aus Niederalkyl, Niederalkoxy, Halogen, Hydroxy und Trifluormethyl substituiert ist, darstellt, $R_2$ Niederalkyl, Niederalkenyl oder Phenylniederalkyl, welches im Phenylteil unsubstituiert oder durch Substituenten ausgewählt aus der Gruppe bestehend aus Niederalkyl, Niederalkoxy, Halogen, Hydroxy und Trifluormethyl substituiert ist, darstellt, $R_3$ Wasserstoff ist und Z Niederalkylen, Niederalkyliden oder Niederalkenylen bedeutet, herstellt.

5. Verfahren gemäss Anspruch 1, dadurch gekennzeichnet, dass man eine Verbindung der Formel I, worin $Ar_1$ unsubstituiertes oder durch Substituenten ausgewählt aus der Gruppe bestehend aus Niederalkyl mit bis und mit 7 C-Atomen, Niederalkoxy mit bis und mit 7 C-Atomen, Halogen mit einer Atomnummer bis und mit 35, Hydroxy, Trifluormethyl und 1-Phenylniederalkoxy mit bis und mit 4 C-Atomen im Niederalkoxyteil substituiertes Phenyl bedeutet, $Ar_2$ einen unsubstituierten oder durch Substituenten ausgewählt aus der Gruppe bestehend aus Niederalkyl mit bis und mit 7 C-Atomen, Halogen mit einer Atomnummer bis und mit 35 und Hydroxy substituierten Phenylen- oder Thienylenrest bedeutet, $R_1$ Wasserstoff, Niederalkyl mit bis und mit 7 C-Atomen, Niederalkenyl mit 3 der 4 C-Atomen oder Phenylniederalkyl, welches bis und mit 7 C-Atome im Niederalkylteil aufweist und dessen Phenylteil unsubstituiert oder durch Substituenten ausgewählt aus der Gruppe bestehend aus Niederalkyl mit bis und mit 7 C-Atomen, Niederalkoxy mit bis und mit 7 C-Atomen, Halogen mit einer Atomnummer bis und mit 35, Hydroxy und Trifluormethyl substituiert ist, darstellt $R_2$ Niederalkyl mit bis und mit 7 C-Atomen, Niederalkenyl mit 3 oder 4 C-Atomen oder Phenylniederalkyl, welches bis und mit 7 C-Atome im Niederalkylteil aufweist und dessen Phenylteil unsubstituiert oder durch Substituenten ausgewählt aus der Gruppe bestehend aus Niederalkyl mit bis und mit 7 C-Atomen, Niederalkoxy mit bis und mit 7 C-Atomen, Halogen mit einer Atomnummer bis und mit 35, Hydroxy und Trifluormethyl substituiert ist, darstellt, $R_3$ Wasserstoff ist und Z Niederalkylen mit bis und mit 4 C-Atomen, Niederalkyliden mit 2 bis und mit 4 C-Atomen oder Niederalkenylen mit 2 bis und mit 4 C-Atomen bedeutet, herstellt.

6. Verfahren gemäss Anspruch 1, dadurch gekennzeichnet, dass man eine Verbindung der Formel I, worin $Ar_1$ durch Niederalkyl mit bis und mit 4 C-Atomen, Niederalkoxy mit bis und mit 4 C-Atomen, Halogen mit einer Atomnummer bis und mit 35, Hydroxy und/oder Benzyloxy substituiertes Phenyl bedeutet, $Ar_2$ einen unsubstituierten oder durch Hydroxy substituierten Phenylen- oder Thienylenrest bedeutet, $R_1$ Wasserstoff oder 1-Phenylniederalkyl mit bis und mit 4 C-Atomen im Niederalkylteil bedeutet, $R_3$ Wasserstoff ist, $R_2$ Niederalkyl mit bis und mit 4 C-Atomen bedeutet und Z Niederalkylen mit bis und mit 4 C-Atomen oder Niederalkenylen mit 2 bis und mit 4 C-Atomen bedeutet, herstellt.

7. Verfahren gemäss Anspruch 1, dadurch gekennzeichnet, dass man eine Verbindung der Formel I, worin $Ar_1$ durch Niederalkyl mit bis und mit 4 C-Atomen, Niederalkoxy mit bis und mit 4 C-Atomen, Halogen mit einer Atomnummer bis und mit 35, Hydroxy und/oder Benzyloxy ein-, zwei-, drei-oder vierfach substituiertes Phenyl bedeutet, $Ar_2$ unsubstituiertes 1,2-, 1,3- oder 1,4-Phenylen oder unsubstituiertes 3,4-Thienylen bedeutet, $R_1$ Wasserstoff, 1-Phenylniederalkyl mit bis und mit 4 C-Atomen im Niederalkylteil oder $C_1$-$C_4$-Alkyl ist, $R_2$ $C_1$-$C_4$-Alkyl darstellt, $R_3$ Wasserstoff oder $C_2$-$C_5$-Alkanoyl ist und Z $C_1$-$C_4$-Alkylen oder $C_2$-$C_4$-Alkenylen bedeutet, herstellt.

8. Verfahren gemäss Anspruch 1, dadurch gekennzeichnet, dass man eine Verbindung der Formel I, worin $Ar_1$ durch Halogen mit einer Atomnummer bis und mit 35 in den Positionen 2 und 6 zweifach substituiertes Phenyl bedeutet, $Ar_2$ 1,2-Phenylen bedeutet, $R_1$ Wasserstoff oder Benzyl bedeutet, $R_2$ Niederalkyl mit bis und mit 4 C-Atomen bedeutet, $R_3$ Wasserstoff ist und Z Methylen oder Ethylen bedeutet, herstellt.

9. Verfahren gemäss Anspruch 1, dadurch gekennzeichnet, dass man eine Verbindung der Formel I, worin $Ar_1$ 2,6-Difluorphenyl oder 2,6-Dichlorphenyl ist, $Ar_2$ unsubstituiertes 1,2- oder 1,4-Phenylen oder

unsubstituiertes 3,4-Thienylen darstellt, $R_1$ Wasserstoff oder Benzyl bedeutet, $R_2$ $C_1$-$C_4$-Alkyl ist, $R_3$ Wasserstoff ist und Z $C_1$-$C_4$-Alkylen bedeutet, herstellt.

10. Verfahren gemäss Anspruch 1, dadurch gekennzeichnet, dass man eine Verbindung der Formel I, worin $Ar_1$ 2,6-Dichlorphenyl ist, $Ar_2$ unsubstituiertes 1,2-Phenylen oder unsubstituiertes 3,4-Thienylen ist, $R_1$ Wasserstoff ist, $R_2$ $C_1$-$C_4$-Alkyl ist, $R_3$ Wasserstoff ist und Z Methylen bedeutet.

11. Verfahren gemäss Anspruch 1, dadurch gekennzeichnet, dass man [o-[(2,6-Dichlorphenyl)-amino]-phenyl]-aceto-N-methyl-hydroxamsäure, herstellt.

12. Verfahren gemäss Anspruch 1, dadurch gekennzeichnet, dass man 4-[o-[(2,6-Dichlorphenyl)-amino]-phenyl]-butyro-N-methyl-hydroxamsäure, herstellt.

13. Verfahren gemäss Anspruch 1, dadurch gekennzeichnet, dass man [4-[(2,6-Dichlor-phenyl)-amino]-thien-3-yl]-aceto-N-methyl-hydroxamsäure, herstellt.

14. Verfahren gemäss Anspruch 1, dadurch gekennzeichnet, dass man [m-[(2,6-Dichlorphenyl)-amino]-phenyl]aceto-N-methyl-hydroxamsäure, herstellt.

15. Verfahren gemäss Anspruch 1, dadurch gekennzeichnet, dass man [p-[(2,6-Dichlorphenyl)-amino]-phenyl]-aceto-N-methyl-hydroxamsäure, herstellt.

16. Verfahren gemäss Anspruch 1, dadurch gekennzeichnet, dass man [o-[(2,6-Dichlor-4-benzyloxy-3-methoxy-phenyl)-amino]-phenyl]-aceto-N-methyl-hydroxamsäure, [o-[(2,6-Dichlor-4-hydroxy-3-methoxy-phenyl)-amino]-phenyl]-aceto-N-methyl-hydroxamsäure, [o-[(2,6-Dichlorphenyl)-N-benzyl-amino]-phenyl]-aceto-N-methyl-hydroxamsäure, [o-(2,6-Dichlorphenyl)-amino]-cinnamo-N-methyl-hydroxamsäure, [o-[(2,6-Difluorphenyl)-amino]-phenyl]-aceto-N-methyl-hydroxamsäure, 3-[o-[(2,6-Dichlorphenyl)-amino]-phenyl]-propio-N-methyl-hydroxamsäure, [o-[(2,6-Dichlor-phenyl)-amino]-phenyl]-aceto-N-ethyl-hydroxamsäure, [o-[(2,6-Dichlor-phenyl)-amino]-phenyl]-aceto-N-isopropyl-hydroxamsäure, [o-[(3,5-Dichlor-phenyl)-amino]-phenyl]-aceto-N-methyl-hydroxamsäure, [o-[(2,6-Dimethyl-phenyl)-amino]-phenyl]-aceto-N-methyl-hydroxamsäure, [o-[(2-Chlor-6-methyl-phenyl)-amino]-phenyl]-aceto-N-methyl-hydroxamsäure oder [o-[(2,6-Dichlor-phenyl)-N-methyl-amino]-phenyl]-aceto-N-methyl-hydroxamsäure, herstellt.

17. Verfahren gemäss Anspruch 1, dadurch gekennzeichnet, dass man [o-[(2,6-Dichlor-3-hydroxy-4-methoxy-phenyl)amino]phenyl]aceto-N-methyl-hydroxamsäure, [o-(2,6-Dichlorphenyl)-N-benzyl-amino]-cinnamo-N-methyl-hydroxamsäure, [o-(2,6-Dichlorphenyl)amino]phenyl]aceto-N-methyl-o-acetyl-hydroxamsäure oder [o-[(2,6-Dichlorphenyl)-amino]-phenyl]-aceto-N-methyl-o-pivaloyl-hydroxamsäure, herstellt.

18. Verfahren gemäss Anspruch 1, dadurch gekennzeichnet, dass man eine Verbindung der Formel I, erhältlich gemäss einem der Ansprüche 1 bis 17, in Form eines Salzes, herstellt.

19. Verfahren gemäss Anspruch 1, dadurch gekennzeichnet, dass man eine Verbindung der Formel I, erhältlich gemäss einem der Ansprüche 1 bis 18, in Form eines pharmazeutisch verwendbaren Salzes, herstellt.

20. Verfahren zur Herstellung eines pharmazeutischen Präparates, dadurch gekennzeichnet, dass man
a) eine Verbindung der Formel

$$Ar_1 - \underset{R_1}{N} - Ar_2 - Z - COOH \qquad (IIa)$$

oder ein Salz und/oder ein reaktionsfähiges Derivat davon mit einer Verbindung der Formel
$R_3$- O - HN - $R_2$ (IIb)
oder einem Salz davon umsetzt oder
b) zur Herstellung einer Verbindung I, worin $R_3$ Wasserstoff ist, oder eines Salzes davon in einer Verbindung der Formel

$$Ar_1 - \underset{R_1}{N} - Ar_2 - Z - CO - \underset{R_2}{N} - OP_1 \qquad (III)$$

oder einem Salz davon die Schutzgruppe $P_1$ in Wasserstoff überführt oder
c) eine Verbindung der Formel

$$Ar_1 - \underset{R_1}{N} - H \qquad (IVa)$$

oder ein Salz davon mit einer Verbindung der Formel

$$X_1 - Ar_2 - Z - CO - \underset{R_2}{N} - O - R_3 \quad (IVb),$$

worin $X_1$ reaktionsfähiges verestertes Hydroxy bedeutet, oder einem Salz davon umsetzt und jeweils gewünschtenfalls eine verfahrensgemäss oder auf andere Weise erhältliche Verbindung I in eine andere Verbindung I überführt, ein verfahrensgemäss erhältliches Isomerengemisch in die Komponenten auftrennt, ein verfahrensgemäss erhältliches Enantiomeren-beziehungsweise Diastereomerengemisch in die Enantiomeren beziehungs weise Diastereomeren aufspaltet und/oder eine verfahrensgemäss erhältliche freie Verbindung I in ein Salz oder ein verfahrensgemäss erhältliches Salz in die freie Verbindung I oder in ein anderes Salz umwandelt und eine auf diese Weise erhältliche Verbindung der Formel

$$Ar_1 - \underset{R_1}{N} - Ar_2 - Z - CO - \underset{R_2}{N} - O - R_3 \quad (I),$$

worin $Ar_1$ einen unsubstituierten oder substituierten Arylrest bedeutet, $Ar_2$ einen unsubstituierten oder substituierten Arylenrest bedeutet, $R_1$ Wasserstoff oder einen aliphatischen oder araliphatischen Rest bedeutet, $R_2$ einen aliphatischen oder araliphatischen Rest bedeutet, $R_3$ Wasserstoff, Niederalkyl oder Niederalkanoyl darstellt und Z einen zweiwertigen aliphatischen Rest bedeutet, in freier Form oder in From eines pharmazeutisch verwendbaren Salzes, gegebenenfalls unter Beimischung von üblichen pharmazeutischen Hilfsstoffen, zu einem pharmazeutischen Präparat verarbeitet.

21. Verfahren zur Herstellung eines pharmazeutischen Präparates dadurch gekennzeichnet, dass man eine Verbindung der Formel I, erhältlich gemäss einem der Ansprüche 1 bis 17, oder ein pharmazeutisch verwendbares Salz davon, erhältlich gemäss Anspruch 19, gegebenenfalls unter Beimischung von üblichen pharmazeutischen Hilfsstoffen, zu einem pharmazeutischen Präparat verarbeitet.

22. Verfahren zur Herstellung eines pharmazeutischen Präparates gemäss Anspruch 21, dadurch gekennzeichnet, dass man eine Verbindung der Formel I, erhältlich gemäss einem der Ansprüche 2, 4 bis 6, 8 und 11 bis 16, gegebenenfalls unter Beimischung von üblichen pharmazeutischen Hilfsstoffen, zu einem pharmazeutischen Präparat verarbeitet.

23. Verfahren gemäss einem der Ansprüche 20 bis 22 zur Herstellung eines antiinflammatorisch und/oder antiallergisch wirksamen pharmazeutischen Präparates, dadurch gekennzeichnet, dass man einen antiinflammatorisch und/oder antiallergisch wirksamen Wirkstoff wählt.

24. Verfahren gemäss Anspruch 22 zur Herstellung eines antiinflammatorisch und/oder antiallergisch wirksamen pharmazeutischen Präparates, dadurch gekennzeichnet, dass man einen antiinflammatorisch und/oder antiallergisch wirksamen Wirkstoff wählt.

25. Verwendung einer Verbindung der Formel I, erhältlich gemäss einem der Ansprüche 1 bis 17 oder eines pharmazeutisch verwendbaren Salzes davon, erhältlich gemäss Anspruch 19, zur Herstellung eines pharmazeutischen Präparats.

26. Verwendung einer Verbindung der Formel I oder eines pharmazeutisch verwendbaren Salzes davon gemäss Anspruch 25, zur Herstellung eines Antiinflammatorikums und/oder Antiallergikums.

27. Verwendung einer Verbindung der Formel I, erhältlich gemäss einem der Ansprüche 1 bis 17 oder eines pharmazeutisch verwendbaren Salzes davon, erhältlich gemäss Anspruch 19, zur Herstellung eines pharmazeutischen Präparats auf nicht-chemischem Wege.